# EUROPEAN PATENT SPECIFICATION

(11) **EP 2 555 800 B1**
(45) Date of publication and mention of the grant of the patent: **21.03.2018**
(21) Application number: 11715125.8
(22) Date of filing: 05.04.2011
(51) Int. Cl.: A61K 41/00, A61K 47/69, A61K 47/64, A61K 47/68, A61K 47/55, A61N 5/10, A61P 35/00, B82Y 5/00

(54) **ENHANCEMENT OF RADIATION THERAPY BY TARGETED HIGH-Z NANOPARTICLES**
VERBESSERTE STRAHLENTHERAPIE ANHAND VON GEZIELTEN NANOPARTIKELN MIT HOHER ORDNUNGSZAHL
AMÉLIORATION D'UNE THÉRAPIE PAR RAYONNEMENT PAR NANOPARTICULES CIBLÉES À Z ÉLEVÉ

(30) Priority: 05.04.2010 US 341819 P
(43) Date of publication of application: 13.02.2013
(73) Proprietor: Nanospectra Biosciences, Inc., Houston, TX 77054 (US); The Board of Regents of the University of Texas System, Austin, TX 78701 (US)
(72) Inventor: KRISHNAN, Sunil, Houston, TX 77030 (US); DIAGARADJANE, Parmeswaran, Houston TX 77054 (US); GOODRICH, Glenn P., Houston TX 77054 (US); PAYNE, J. Donald, Houston TX 77054 (US)
(74) Representative: Tomkins & Co
(86) International application number: PCT/US2011/031262
(87) International publication number: WO 2011/127061

(56) References cited:
- US-A1- 2004 181 114
- US-A1- 2010 034 735
- LI X ET AL: "Enhancement of cell recognition in vitro by dual-ligand cancer targeting gold nanoparticles", BIOMATERIALS 2011 ELSEVIER LTD GBR LNKD- DOI:10.1016/J.BIOMATERIALS.2010.12.031, vol. 32, no. 10, April 2011 (2011-04), pages 2540-2545, XP002638705, ISSN: 0142-9612
- CHATTOPADHYAY NILADRI ET AL: "Design and Characterization of HER-2-Targeted Gold Nanoparticles for Enhanced X-radiation Treatment of Locally Advanced Breast Cancer", MOLECULAR PHARMACEUTICS, vol. 7, no. 6, November 2010 (2010-11), pages 2194-2206, XP002638706, ISSN: 1543-8384
- LU W ET AL: "Targeted photothermal ablation of murine melanomas with melanocyte-stimulating hormone analog - conjugated hollow gold nanospheres", CLINICAL CANCER RESEARCH 20090201 US LNKD- DOI:10.1158/1078-0432.CCR-08-1480, vol. 15, no. 3, 1 February 2009 (2009-02-01), pages 876-886, XP002638707, ISSN: 1078-0432 cited in the application

## Description

### FIELD OF THE INVENTION

The present invention relates generally to the field of radiation therapy, and more specifically to high-Z nanoparticles according to the claims for use in radiation therapy for the treatment of cancer.

### BACKGROUND

Radiation therapy is a long-established and effective component of modem cancer therapy for localized disease. However, the ultimate utility of radiation therapy is limited by the fact that some cancer cells are resistant to ionizing radiation. Additionally, the delivery of the ionizing radiation through healthy tissue or beyond the tumor margin limits the radiation dose and may result in unwanted side effects. Attempts to improve outcomes of radiation therapy have largely focused on (i) increasing the dose of radiation delivered to the tumor while minimizing radiation to healthy tissue, (ii) sensitizing the radio-resistant fraction of tumor cells to conventional doses of radiation, and (iii) targeting cancer cells specifically while administering radiation therapy. The present invention uses nanotechnology-based techniques that combine these three approaches to improve radiation therapy outcomes for cancer. Nanoparticles comprised of high atomic weight (high-Z) elements are allowed to specifically accumulate in the target of radiation therapy, providing a localized dose enhancement as a result the interaction of the high atomic number (Z) element with the incident radiation. The selectivity of the nanoparticle for the target tissue allows the radiation dose to be enhanced at the target.

### Radiation dose enhancement using high-Z nanoparticles

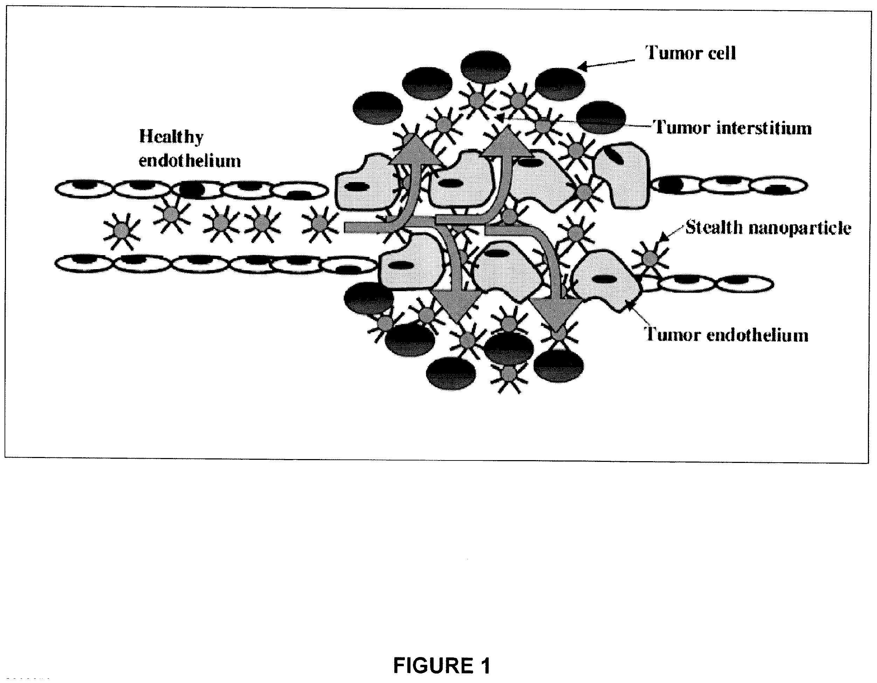

In recent years, intravenously administered nanoparticles (NPs) have shown great promise as anti-cancer agents. These NPs accumulate preferentially within tumors largely as a result of their size and passive extravasation from the leaky, chaotic and immature vasculature of tumors - a phenomenon referred to as the "enhanced permeability and retention" (EPR) effect (Dvorak, el al. 1988; Unezaki, et al. 1996; Maeda, et al. 2003) These nanoparticles may serve individually as therapeutic agents or serve as a carrier for drugs or toxins to effect therapy. In order to increase accumulation of the NP in the tumor, "'stealth agents (e.g. Polyethylene Glycol, or PEG) may be used to reduce the immunogenicity of the NP. The NP may also be "targeted" using a ligand that will bind to the surface of the target cell. See Figure 1 for an illustration of the EPR effect. In order to use the EPR effect for tumor accumulation, the NP must be within a size range to reduce extravasation into non-tumor areas but also allow accumulation through the EPR effect. In general, a NP less than 5 nm in diameter (or in its longest dimension) may accumulate in tissues through mechanisms unrelated to the EPR effect, either through pinocytosis or extravasation through the juncture between endothelial cells. Additionally, a NP less than 5.5 nm in diameter (or its longest dimension) may be cleared from the blood through the kidneys, reducing its availability for accumulation in a tumor.(Choi, et al. 2007) On the other hand, NPs greater than 200-400 nm are unlikely to accumulate through the EPR because the NP exceeds the size of the fenestrations in the tumor. However, NP greater than 200 nm may be used to target the vasculature of tumor cells because extravasation from the blood stream may not be required.

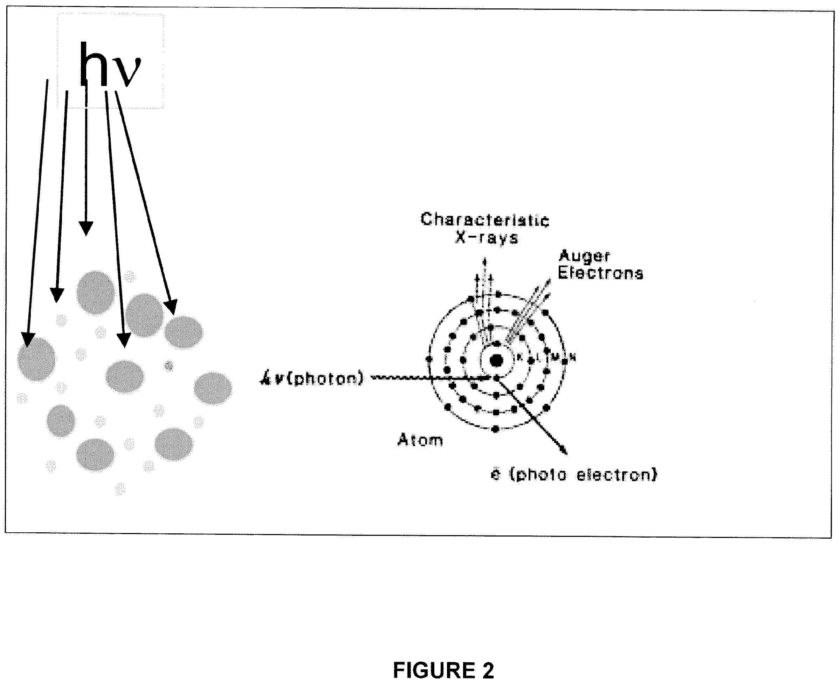

When an element is exposed to radiation, the incident energy may be absorbed by an electron within the element and the electron ejected from its orbit. This is illustrated in Figure 2. If this electron is an inner-shell electron, the hole left behind by its ejection is filled by electrons that drop down from outer orbits - the resulting transition in binding energies of that electron result in the release of characteristic X-rays that are unique to the metal being irradiated (i.e., the photoelectric effect). More important!}, the probability of photoelectron interaction with tissue resulting in radiation dose deposition within it is a function of the atomic number of the metal and the incident photon energy (in fact that relationship is a function of Z³⁴ where Z is the atomic number). Consequently, artificially increasing the atomic number of tumor tissues will increase radiation dose deposition within them.

Until the present invention, dose enhancement effect from high-Z elements has not been demonstrated in a tumor-selective manner. Previously, the passive EPR targeting strategy was used by Hainfeld et al (Hainfeld et al. 2004) to demonstrate that irradiation of tumor-bearing mice immediately after injection with 1.9nm diameter gold nanoparticles (GNPs) results in tumor regression and long-term survival (1 year survival rate of 86%) without any significant toxicity compared to mice irradiated without GNPs (1 year survival rate of 20%>). This outcome was attributed to an increase in photoelectric absorption due to the high Z of gold followed by the greater physical damage to tumor cells and endothelial cells lining the blood vessels by photo and Auger/Coster- ronig electrons from GNPs.

The effects observed by Hainfeld et al were observed only when extremely large quantities of gold were infused (injected GNP concentration of 2.7 g/Kg; injection volume of 10 µí̈/g body weight) and when the infusion was immediately (2 min) followed by irradiation. At these early time points, the tumor gold content is largely an index of the vascularity of the tumor, with GNPs serving merely as contrast agents. It is expected that small particles of this size will be cleared from the blood by the kidneys, resulting in more rapid excretion from the body. The requisite gold content in the tumor (7 mg gold per gram of tumor), the timing of radiation (2 min after injection) and the radiation source (250 kVp) make this approach clinically less appealing. Additionally, the size of the nanoparticle used does not allow for tumor selectivity of the enhancement; as indicated b Hainfeld, there was a high background level of gold in tissue outside of the tumor.

The US patents of Hainfeld, et al (US Nos. 6.955.639, 7, 194.063, 7,367,934, 7.530,940 and US application 12/415,618) all teach that radiation dose enhancement is achieved if the gold accumulation in tumor is preferably 0.3% or more by mass of the tumor, but at least 0.05% by mass. The injected dose of gold required for that accumulation was approximately 1 gram of gold per kilogram of body weight (1 part per thousand). This process required an extremely high dose given the current cost of gold and the costs of manufacturing the NP, and has not been commercialized. These high doses also exceed previous uses of gold in therapy, and raise questions regarding toxicity, particularly with re-dosing for subsequent treatments. Generally only a fraction of the gold dose injected into the blood stream accumulates in a tumor, with the remainder cleared from the blood stream by the reticuloendothelial system To achieve the gold accumulations in the tumor taught by Hainfeld requires an extremely large dose of gold injected into the blood stream, much of which will be cleared from the blood by the liver and spleen or, if the gold particle is sufficiently small, cleared through the kidney.

Additionally, the radiation effects were observed by Hainfeld using a single dose of 26 Gy, 250 kVp.
US patent publication number 2004/181114 A1 discloses methods of using gold nanoparticles to enhance the dose and effectiveness of x-rays and other kinds of radiation in ablating a target tissue such as a tumour. The gold nanoparticles have a thioglucose shell. US patent publication number 2010/0034735 A1 discloses modified gold nanoparticles that enable a non-invasive, real-time, targeted cancer imaging-therapeutic in one step. These particles have a cysteamine or glucose coating.

### SUMMARY

High-Z particles for use in a method for enhancing the effects of radiation directed to a tissue or a population of cells, characterized in that the high -Z particles are gold nanorods and have a surface coating of polyethylene glycol (PEG);
the high-Z particles comprise a targeting molecule with an affinity for a targeted tissue or a targeted population of cells and a high-Z element;
the high-Z particles are administered to an animal and the targeted tissue or targeted population of cells is subsequently irradiated with ionizing radiation;
wherein the high-Z particles are administered to the animal in an amount sufficient to achieve a concentration in the targeted tissue or the targeted population of cells of less than 0.05% metal by weight.

The present invention relates to the design and manufacturing of a high-Z particle for use in enhancing the effects of ionizing radiation. The localization of a high-Z particle near the nucleus of a target cell will enhance the effect of ionizing radiation and increase DNA strand damage, resulting in a therapeutic benefit. In particular, the use of a targeting molecule to enable cellular uptake by the target cells (tumor cells or endothelial cells proximate to the tumor) will enhance the dose effect.

In one embodiment, the ionizing radiation is directed to a target cell or tissue using external beam radiation, including intensity modulation or conforming beam methods. In another embodiment, the ionizing radiation is delivered intratumorally by brachytherapy seeds or other methods. In a separate embodiment, the source of radiation may be protons or other charged particles.

In one embodiment, the energy of the radiation source is above the K-edge of the high-Z NP. In another embodiment, the energy of the radiation source is not selected based on the -edge of the high-Z P.

In another embodiment, the target is exposed to ionizing radiation in a continuous flow extracorporeal device.

In the various embodiments, the targeting molecules may be selected from among antibodies, antibody fragments, peptides, proteins, aptamers, oligonucleotides or other molecules.

In this invention, the particle dose is several orders of magnitude less than the doses previously used to achieve a radiation dose enhancement. This is principally the result of timing the irradiation to match the uptake of the particle in the tumor and using a targeting molecule free of steric hindrance to result in longer tumor retention as well as cellular uptake. In one embodiment, the particle dose that accumulates in the tumor is less than 0.05% by mass of the target tissue. In one embodiment the particle dose administered parenterally in each administration is less than 0.05% by mass of the animal mass.

In this invention, gold nanorods are used as targeted high-Z particles.

In one embodiment, the NP is administered intravenously. In another embodiment, the NP is administered into the lymphatic system. In another embodiment the NP is directly injected into the tumor.

In one embodiment, the NP is less than 400 nm and greater than 8 nm along its longest dimension. In another embodiment, the NP is preferably greater than 10 nm and less than 200 nm along its longest dimension. In another embodiment. The NP is preferably greater than 20 nm and less than 100 nm along its longest dimension.

The NP surface is conjugated with a polymer to increase circulation time in the blood stream. The polymer is a polyethylene glycol.

The NP is rod-shaped. In an embodiment, the high-Z element may be contained in a liposome or micelle.

In another embodiment, the NP is comprised of a high-Z element and an anti-sense oligonucleotide.

In one embodiment, the NP is comprised of a high-Z element and a chemotoxic agent. In one embodiment, the chemotoxic agent is tumor necrosis factor. In another embodiment, the chemotoxic agent is paclitaxel.

In one embodiment, the target cells or tissue are cancers. In another embodiment, the target cells or tissue are vascular malformations.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
FIGURE 1 shows an illustration of passive accumulation through the "enhanced permeability and retention effect", or EPR. From Brigger et al, Adv. Drug Deliv. Rev. 54, 2002, Nanoparticles injected intravenously and designed to circulate in the blood stream for a predetermined period of time (stealth nanoparticles) will accumulate in a tumor through the leaky vasculature of the tumor;
FIGURE 2 shows an overview of photoelectric effect, one method of interaction between X-ray radiation and elements, the source of radiation dose enhancement to tumors. When an element is exposed to radiation, the incident energy may be absorbed by an electron within the element and the electron ejected from its orbit. If this electron is an inner-shell electron, the hole left behind by its ejection is filled by electrons that drop down from outer orbits - the resulting transition in binding energies of that electron result in the release of characteristic X-rays that are unique to the metal being irradiated. More importantly, the probability of photoelectron interaction with tissue resulting in radiation dose deposition within it is a function of the atomic number of the metal and the incident photon energy (in fact, that relationship is a function of Z³⁻⁴ where Z is the atomic number). Consequently, artificially increasing the atomic number of tumor tissues will increase radiation dose deposition within them;
FIGURE 3 shows an illustration of the method of manufacture of one embodiment, a cetuximab-labeled gold nanorod (C-GNR);

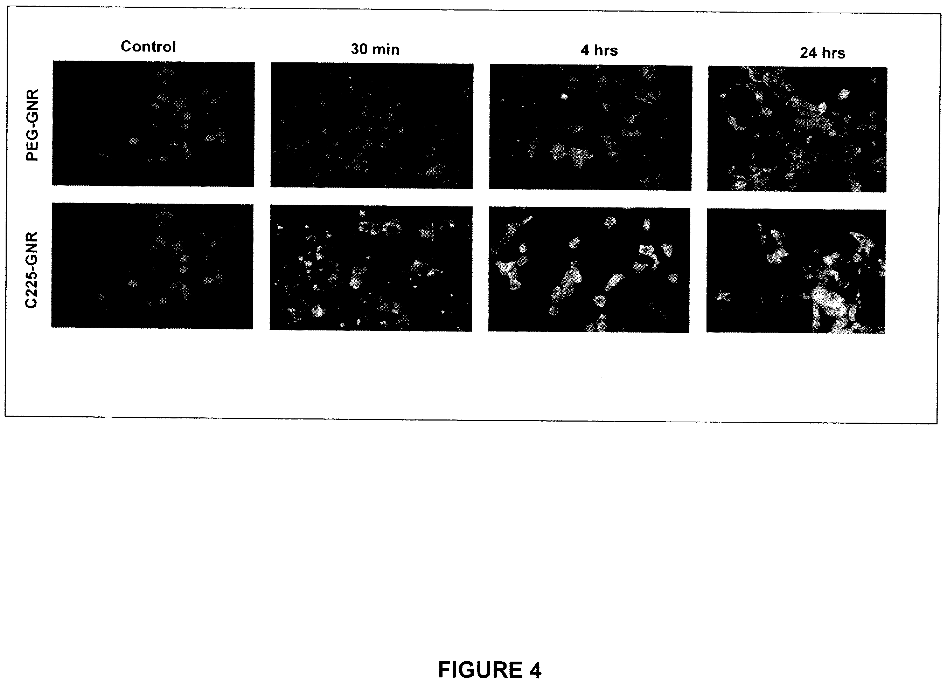

FIGURE 4 shows an illustratiuon that the C225 (cetuximab) targeted GNR have a binding affinity for the HCT116 cells. In vitro validation of the conjugates was evaluated in HCT116 cells grown on a microscopic cover slip and incubated for 30 min with 500µl of PEG-GNR and C-GNR (2x1011 GNR/ml). Cells were stained with 1 µM DAPI (nuclear marker), fixed in 2% paraformaldehyde and visualized by dark field microscopy at different time intervals after treatment. As noted, greater accumulation and internalization of conjugated GNRs was observed at 24 hrs than at earlier time points. When compared to the PEG-GNRs, C-GNRs displayed excellent cell-surface binding, which was blocked by cetuximab pre-treatment;
FIGURE 5 shows tumor re-growth delay assay with HCT116 xenografts;

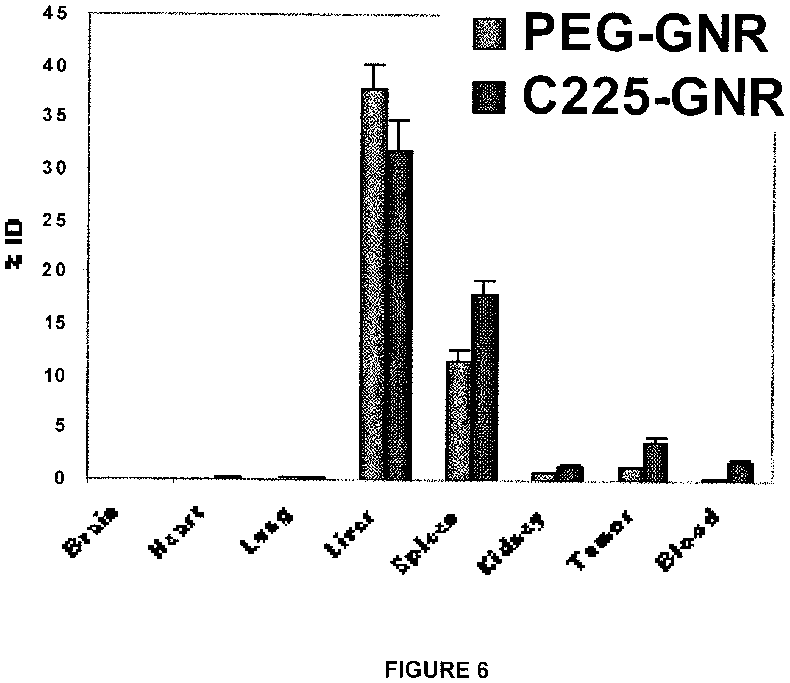

FIGURE 6 shows inductively coupled plasma - mass spectrometry (ICP-MS) analysis of biodistribution of GNRs and conjugated GNRs 24 hrs after intravenous injection in nude mice beraing HTC116 tumors. Individual organs harvested 24 hrs after injection of ∼2x1011 GNRs or C-GNRs via the tail vein to mice bearing HCT116 xenografts. By injecting less than 0.0002% by mass of the body weight, tumor gold accumulation was less than 0.00007% by mass of the tumor, i.e., substantially lower than the gold accumulation typically believed necessary for dose enhancement. C-GNRs accumulated more within tumors than GNRs;
FIGURE 7 shows Dose Enhancement Factor (DEF) as modeled. See Cho SH. Phys Med Biol 2005; 50: N163-73. The models indicate that a tumor gold content (the high-Z element modeled in this case) of >0.7% (7mg/gram of tumor) was required to achieve a DEF of greater than 5%;

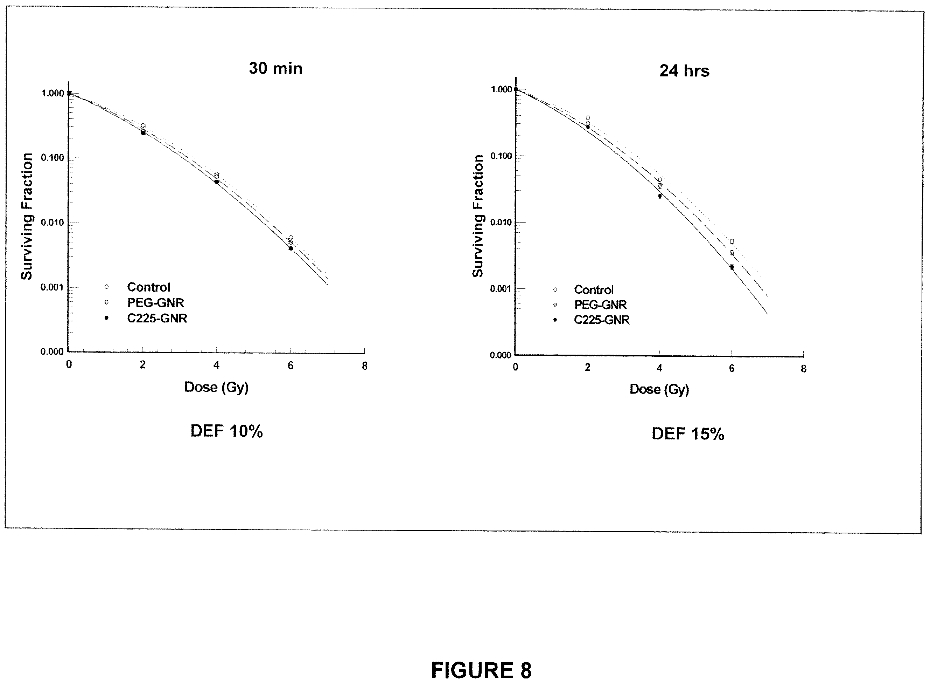

FIGURE 8 shows clonogenic survival of HCT116 cells exposed to unconjugated and conjugated GNRs suggest that a dose-enhancement of 10% can be achieved at 30 mins and 15% at 24 hrs following a single dose of kilovolt (250 kVp) radiation. Data is typically represented as a log-linear curve with the percentage of surviving colonies of cancer cells represented on a log scale. Consequently, the separation of the curves noted in these figures is substantial and clinically meaningful, particularly since this is a modeling of a single radiation dose. Extrapolating these data to typical multiple-fraction radiation treatments for cancer (typically around 25 treatments), the single-fraction dose enhancement factor of 1.15 would represent a (1.15)²⁵ cumulative dose enhancement in an idealized situation;
FIGURE 9 shows γ-H2AX foci at 1 an 4 hrs following radiation (shaded area=nucleus, brighter spots= γ-H2AXfoci);
FIGURE 10 shows quantitative representation of γ-H2AX foci observed in HTC116 cells;
FIGURE 11 shows Western blot analysis of γ-H2AX in nuclear extracts of HCT116 cells;
FIGURE 12 shows a Western blot analysis of γ-H2AX and increased DNA damage signaling downstream in the HTC116 tumors extracted. No difference was noted in DNA repair proteins. This increased DNA damage was also evident in tumor tissues 24 hrs after radiation;
FIGURE 13 shows (Top Panel): Western blot analysis of nuclear extracts in vitro confirmed the increase in DNA damage signaling downstream of the strand breaks (pATM, ATR and chk2 being the sensors and signaling molecules that are downstream of gamma H2AX). (Bottom Panel): Western blot analysis of cytoplasmic extracts, compared to the radiation + GNR group, indicating an increase in bcl2/bax ratio without a change in caspases in the radiation + C-GNR group, which indicates a change in mitochondrial membrane potential;
FIGURE 14 shows that the effect of radiation on DNA was amplified in the presence of conjugated GNRs compared to unconjugated GNRs, there was a difference in mitochondrial redox potential. Under physiological conditions, the equilibrium between NADP and NADPH favors the formation of NADPH but during oxidative stress, there is a shift in this equilibrium towards greater formation of NADP.
FIGURE 15 shows irradiation in the presence of conjugated GNRs resulted in greater oxidative modification of proteins globally than irradiation in the presence of unconjugated GNRs. The protein carbonyl assay measures the covalent modification of proteins caused directly by reactive oxygen species (ROS) or by by-products of oxidative stress. This serves as a stable marker of oxidative modification of proteins detectable immediately in response to ROS-induced oxidative stress;
FIGURE 16 shows a substantial reduction in markers of tumor vascularity when tumors were radiated after treatment with conjugated GNRs compared to unconjugated GNRs;
FIGURE 17 shows average microvessel density;
FIGURE 18 shows TEM imaging showing the progressive internalization of C-GNR over time;
FIGURE 19 shows TEM analysis of C-GNR in tumor tissue; and
Figure 20 shows TEM analysis of C-GNR in tumor tissue.

### DETAILED DESCRIPTION OF PREFERRED EMBODIMENTS

The present invention relates generally to the field of radiation therapy, and more specifically to high-Z nanoparticles for use in radiation therapy for the treatment of cancer.

Initial Monte Carlo studies of GNP-based radiation dose enhancement demonstrated that the macroscopic (or average) tumor dose enhancement depends on the gold concentration within the tumor and the photon beam quality, ranging from several hundred percent for diagnostic x-rays to a few percent for typical clinical megavoltage photon beams.(Cho, 2005) For instance, the dose enhancement for a superficial tumor treated with 140 kVp x-rays could be at least a factor of 2 at a gold concentration of 7 mg Au /g tumor (7 parts per thousand). As predicted by subsequent Monte Carlo models (Cho, et al. 2009), to maximize the dose enhancement, the ideal source would have a dominant energy spectrum just above the K-edge of gold (80.7 keV) like ¹⁶⁹Yb (half-life = 32.0 days, intensity-weighted average gamma ray energy ∼93 keV) brachytherapy source or the L-edge of gold (∼10 keV) like the classical brachytherapy sources ¹⁰³Pd and ¹²⁵I or the low energy 50 kVp x-ray source. In all of these instances, the efficacy of treatment depends upon the sustained presence of GNPs within the tumor but not within the adjacent normal tissue.

### Active targeting of Nanoparticles to Tumor Cells

The present invention relates to tumor-specific accumulation of systemically administered nanoparticles using conjugation of NPs to molecular markers specifically expressed on the surface of tumor cells, otherwise referred to as 'active targeting'. While the portion of the total NP dose injected into the blood that accumulates in the tumor may not be materially affected by the addition of the targeting molecule, the distribution within and retention of the gold particle in the tumor may be affected by these targeting molecules. (Huang, et al.)

The selection of a targeting ligand is dependent on the targeting objectives. In general, NP enter a tumor through the EPR. Once in the tumor, the targeting ligand may be used to increase retention in the tumor and reduce lymphatic clearance. Alternatively, the targeting ligand may be selected to result in internalization of the NP within the tumor cell, which generally requires binding or affinity with a cell surface molecule. In certain cases, if the tumor vasculature is the target of therapy, the targeting ligand may be chosen to result in binding or affinity with a cell surface ligand, and may be selected to result in internalization within the endothelial cell.

Alternatively, the NP may be designed to enhance internalization within the tumor cell, which may be affected by the shape or surface charge of the particle.

With respect to cell surface molecules that may be targets, it is known that certain tumor cell surface targets may result in internalization of the NP within the cell. Additionally, internalization may be induced or affected by the NP chosen. Among the tumor and tumor-vasculature related targets are the epidermal growth factor receptor, human epidermal growth factor receptors 2-4, the folate receptor, the melanocyte stimulating hormone receptor, the vascular endothelial growth factor receptors, and the integrins, insulin-like growth factor receptor, hepatocyte growth factor receptor, and basic fibroblast growth factor receptor. The targeting ligands to these receptors include antibodies (or portions thereof), peptides, aptamers, proteins, and other molecules.

Among a wide array of tumor-specific biomarkers, the epidermal growth factor receptor (EGFR) is increasingly viewed as a viable therapeutic target because it is ubiquitously over-expressed in a wide variety of cancers and drives their unchecked growth and proliferation, invasiveness, angiogenic and metastatic potential, and resistance to traditional cancer therapies.(Blume-Jensen and Hunter 2001; Baselga 2006) Specific targeting of this receptor using a humanized monoclonal antibody (cetuximab) improves patient survival in a variety of clinical situations.(Goldberg, 2005) In particular, the efficacy of combination of cetuximab and radiation in head and neck cancer led to the first and only approval of a targeted therapy combination with radiation therapy.(Bonner, et al. 2006)

Gold nanoparticles (GNP) include nanorods, nanoshells, gold colloids, nanocages, nanoprisms, and other geometries. Key considerations in the selection of the particle geometry include the intravenous circulation kinetics and the particle uptake by the tumor and target cells. Gold is particularly useful because of its biocompatibility.

Currently, across a broad spectrum of GNP geometries that are readily synthesized, gold nanorods (GNRs) offer a few unique characteristics. They are highly stable, cylindrical, solid gold nanoparticles with sufficient gold content to elicit potent radiation dose enhancement. They also absorb strongly in the near infrared (NIR) wavelengths to generate intense heat by photothermal activation of their surface plasmons.(Huang, et al. 2007) When administered systemically in mice via the tail vein GNRs accumulate passively in tumors via the EPR effect and, being strong metallic conductors, deliver this photothermal energy directly and efficiently to tumors. Tumor uptake can be enhanced by evading reticuloendothelial capture via surface coating with polyethylene glycol (PEG) or due to their shape.(Caliceti and Veronese 2003; Mitragotri and Lahann 2009) The PEG coating also allows further functionalization with peptides, antibodies and oligonucleotides decorating the surface as well. *In vitro,* the cylindrical shape of GNRs enhances their internalization into cells.(Huff, et al. 2007; Hauck, et al. 2008) *In vivo,* their cylindrical shape leads to their marginalization to the vascular endothelial surface rather than the center of a parabolic advancing front of blood within a blood vessel, allowing greater extravasation from a blood vessel exhibiting laminar flow and more so from a vessel exhibiting turbulent flow.(Lee, Ferrari et al. 2009) Lastly, as with other GNPs, favorable characteristics of GNRs used in clinical applications are the lack of biochemical reactivity, inertness, clinical safety record of gold used for decades in the treatment of arthritis, and the relative ease and lower cost of advancing its clinical translation as a device rather than as a drug. Although GNRs are efficient photothermal activators, this property is not directly invoked in all embodiments of this method. Nonetheless, this property could certainly be exploited for specific clinical scenarios such as during intraoperative radiation therapy or for triggered release of chemotoxic agents. The surface of GNRs is similar to other GNPs, and the use of PEG and/or targeting molecules for GNR will be substantially similar for other GNPs.

### Radiation response modulation through radiation dose enhancement and active targeting

The data presented in this application indicates that 'active targeting' of EGFR in a cetuximab-resistant xenograft, in this instance the HCT116 colorectal cancer xenografts, using cetuximab-conjugated GNRs results in remarkable in vivo radiosensitization even when the concentration of GNPs (i.e., GNRs in our study) within tumors is considerably lower than that previously felt to be necessary for radiosensitization based on the work of Hainfeld or the theoretical work of Cho. (Hainfeld, et al. 2004; Cho, 2005; Cho, et al. 2009; Cho, Vassiliev et al. 2007) Stated differently, the likelihood of significant radiosensitization would not be predicted purely from the macroscopic radiation dose enhancement estimates - nevertheless, this was observed. This is best explained by the fact that microscopic dose enhancement estimated at the nano-/cellular-scale provides a better correlation with radiosensitization. From a therapeutic perspective, our data suggest that active targeting leads to radiosensitization in vitro and a reduction in repair of radiation-induced DNA double-strand breaks. From a tumor-targeting perspective, since active targeting with cetuximab-conjugated GNRs overcomes the inherent treatment resistance of HCT116 tumors to traditional targeted therapy with cetuximab.(Manning, et al. 2008) it is likely that the GNR serves as a vector for sustained and concentrated accumulation of cetuximab within the tumor. Indeed, the use of nanoparticles as vectors for delivery of therapeutic payloads has been the subject of intense research for many years.(Pissuwan, et al. 2009) However, here we observe the unique dual functionality of conjugated GNRs where the targeting antibody delivers the GNR to the vicinity of the tumor cell for localized radiation dose enhancement and the GNR delivers the antibody to the tumor cell for enhanced traditional targeted therapy.

These observations demonstrate that non-invasive radiation response modulation may be achieved using targeting molecules and high-Z particles, such as the cetuximab-conjugated GNRs, that can be widely applied as a class solution across multiple tumor types over-expressing the target receptor. For example, EGFR is widely expressed in several cancers. Alternatively, the folate receptor is expressed on many cancer cells, and the particle may be targeted using folic acid. Alternatively, the melanocyte stimulating hormone receptor is expressed on melanoma cells, and the particle may be targeted using the melanocyte stimulating hormone. Alternatively, the integrins alpha-v beta-3 is expressed on the endothelial cells of tumors and on certain cancer cells, and the particle may be targeted using the peptide cyclic-RGD or one of its analogues. Alternatively, the HER-2 receptor is expressed on certain breast and ovarian cancers, and the particle may be targeted with an anti-HER-2 antibody or fragment. Similar results may be achieved using any or a combination of targets.

This invention addresses a critical barrier to the clinical applicability of high-Z (including GNP) mediated radiation response modulation - the inability to attain sufficient intratumoral particle concentration via intravenous administration of unconjugated particles. As demonstrated by the examples, by localizing GNRs in close proximity (including intracellularly) to cancer cells and endothelial cells by active targeting, substantial radiosensitization is possible with much lower intratumoral gold concentrations.

This invention shifts the current research paradigms for cancer therapy using GNP-mediated radiation response modulation. Whereas active targeting does not substantially increase intratumoral concentration of the high-Z material, it improves the efficiency of high-Z-mediated radiation response modulation largely by microscopic radiation dose enhancement at the nano-/cellular-scale, delaying DNA strand break repair, and increasing the biological effectiveness of radiation (similar to high linear-energy transfer radiation therapy).

Also, this serves as a platform for future tumor-specific delivery of a therapeutic payload - i.e., this is a novel approach that simultaneously targets a tumor cell and overcomes its resistance to therapy.

The present invention relates to the design and manufacturing of a high-Z particle for use in enhancing the effects of ionizing radiation. The localization of a high-Z particle near the nucleus of a target cell may enhance the effect of ionizing radiation and increase DNA strand damage, resulting in a therapeutic benefit. In particular, the use of a targeting molecule to enable cellular uptake by the target cells (tumor cells or endothelial cells proximate to the tumor) will enhance the dose effect.

It is important that the targeting molecule be attached in a manner that allows access to the receptor on the target cell. For example, this may be accomplished with the attachment of the targeting molecule through a bi-functional polyethylene glycol chain. Steric hindrance of the targeting molecule should be avoided by proper selection of the linking method.

In one embodiment, the ionizing radiation is directed to a target cell or tissue using external beam radiation, including intensity modulation or conforming beam methods. In another embodiment, the ionizing radiation is delivered intratumorally by brachytherapy seeds or other methods. In a separate embodiment, the source of radiation may be protons or other charged particles.

In one embodiment, the energy of the radiation source is above the K-edge of the high-Z NP. In another embodiment, the energy of the radiation source is not selected based on the K-edge of the high-Z NP.

In another embodiment, the target is exposed to ionizing radiation in a continuous flow extracorporeal device. For example, circulating tumor cells may be targeted by the high-Z particle by injecting the particle into the blood stream, allowing a time delay for uptake by the tumor cells. The blood may then be circulated through an extracorporeal device and exposed to ionizing radiation in the device, and then the blood reinjected into the blood stream, all in a continuous loop.

In the various embodiments, the targeting molecules may be selected from among antibodies, antibody fragments, peptides, proteins, aptamers, oligonucleotides or other molecules. The selected targeting molecules should have an affinity for a cell-surface receptor on the target cells and result in internalization of the particle within the target cell. In one embodiment, the targeting molecule has an affinity for the epidermal growth factor receptor. In another embodiment, the targeting molecule has an affinity for the human epidermal growth factor receptor 2, 3 or 4. In other embodiments the targeting molecule has an affinity for the folate receptor or the melanocyte stimulating hormone receptor.

Radiation dose enhancement is increased when the high-Z NP is closest to the DNA of the target cell. This is first accomplished by selection of a targeting molecule or NP with properties (such as shape or charge) that are internalized by the cell. Additional targeting molecules may be conjugated to the particle that will allow transit through the nuclear membrane. Alternatively, this may be accomplished through the use of a single or multiple targeting molecules.

The targeted particle that accumulates with the tumor is expected to persist longer in the tumor because the targeting molecule enables cellular uptake. As a result, the dose enhancement effect may be achieved through a series of sequential irradiations, a practice common in radiation therapy today. Alternatively, subsequent doses of the targeted particle may be administered during the course of radiation to maintain a dose enhancement effect during the course of radiation treatments.

In this invention, the particle dose is several orders of magnitude less than the doses previously used to achieve a radiation dose enhancement. This is principally the result of timing the irradiation to match the uptake of the particle in the tumor and using a targeting molecule free of steric hindrance to result in longer tumor retention as well as cellular uptake. In one embodiment, the particle dose that accumulates in the tumor is less than 0.05% by mass of the target tissue. In one embodiment the particle dose administered parenterally in each administration is less than 0.05% by mass of the animal mass.

In this invention, gold nanorods are used as targeted high-Z particles. Key considerations in the selection of the particle geometry include the intravenous circulation kinetics and the particle uptake by the tumor and target cells. Gold is particularly useful because of its biocompatibility.

In one embodiment, the NP is administered intravenously. In another embodiment, the NP is administered into the emphatic system. In another embodiment the NP is directly injected into the tumor.

In one embodiment, the NP is less than 400 nm and greater than 8 nm along its longest dimension In another embodiment, the NP is preferably greater than 10 nm and less than 200 nm along its longest dimension. In another embodiment. The NP is preferably greater than 20 nm and less than 100 nm along its longest dimension.

The NP surface is conjugated with a polymer to increase circulation time in the blood stream. The polymer is a polyethylene glycol.

The NP is rod-shaped. In an embodiment, the high-Z element may be contained in a liposome or micelle.

In one embodiment, the NP is comprised of a high-Z element and a chemotoxic agent. In one embodiment, the chemotoxic agent is tumor necrosis factor. In another embodiment, the chemotoxic agent is paclitaxel.

### EXAMPLE 1

The present disclosure indicates that the delivery of NPs to a tumor and the subsequent cellular uptake may result in a significant enhancement of the subsequent radiation dose. As demonstrated, this may be accomplished when the NP content of the tumor is less than 0.05% by mass.

In addition to the specific examples provided here, one can see that this method may be used in a range of cancer types (colorectal, brain, lung, breast, head and neck, pancreatic, ovarian, prostate, melanoma, etc.). Additionally, this method may be used to enhance the radiation delivered to circulating tumor cells, in particular if such cells are exposed to such radiation in an extracorporeal device. Additionally, this method may be used in any clinical application in which the radiation dose is prefereably enhanced.

### EXAMPLE 2

### COLORECTAL CANCER

In colorectal cancers, cetuximab improves overall and progression-free survival in patients with metastatic disease(Van Cutsem, et al. 2009; Cunningham, et al. 2004) Although most tumors overexpress EGFR, not all tumors respond to cetuximab. Two theories have been posited to explain this lack of uniformity of response - either the cetuximab is unable to attain sufficient concentrations at the tumor cell (Saltz, et al. 2004) or the cells have constitutive activating K-ras mutations (downstream of EGFR) that render them insensitive to anti-EGFR therapy (Karapetis, et al. 2008) Although EGFR-targeted therapy has shown promise or proven efficacious in multiple cancer types, the focus of this proposal is on colorectal cancers. Radiation therapy is an essential component of preoperative treatment for locally advanced disease but it results in complete pathological response in only about 20% of patients.(Krishnan, et al. 2006) Adding cetuximab to standard chemoradiation did not improve this response rate, possibly due to the reasons mentioned above.(Rodel, et al. 2008) Radiation is also used as a solitary treatment modality for low-lying rectal cancers in patients who cannot undergo surgery - a 50 kVp endorectal applicator is used in these instances.(Papillon, 1990) In case of recurrent rectal cancer, preoperative radiation is combined with surgery and intraoperative radiation to maximize the chances of cure(Haddock, et al. 2001) - lower energy x-rays are used preoperatively since they deposit more dose near the skin where rectal cancers tend to recur and an ¹⁹²Ir applicator is frequently used intraoperatively.(Beddar, et al. 2006) These clinical scenarios provide the framework for radiation response modulation using NPs that we have developed.

### FABRICATION AND CONJUGATION OF TARGETED GNRS

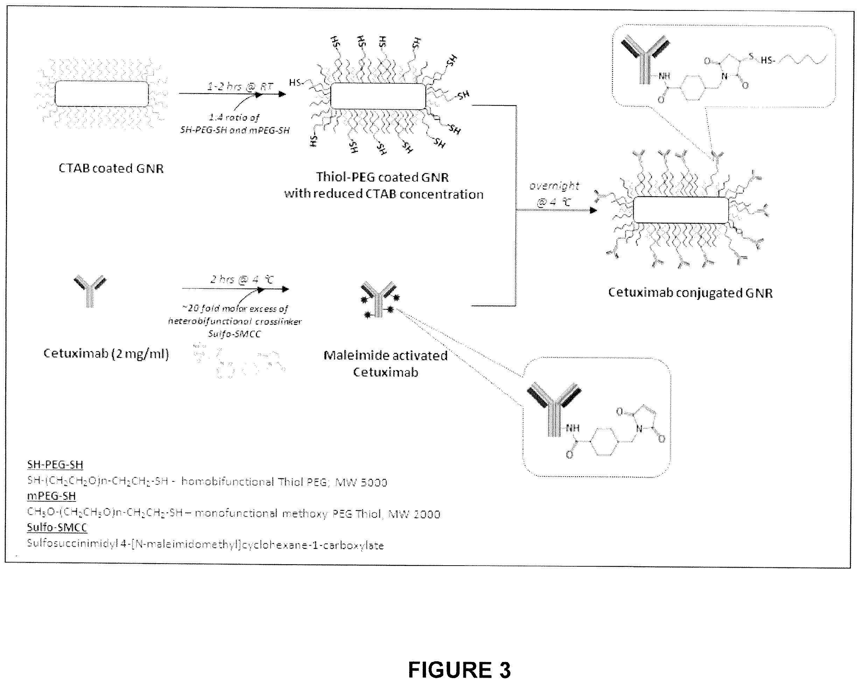

We have synthesized GNRs of varying lengths and aspect ratios using a seed-mediated, surfactant-assisted growth method using published procedures (Jana, et al. 2001; Zharov, et al. 2005) and optimized an aspect ratio of 3.4 (13 nm diameter and 44 nm length) for NIR activation. The hexadecylcetyltrimethyl ammonium bromide (CTAB) coating was replaced with PEG to make them more biocompatible and to prevent nonspecific reticuloendothelial capture. GNRs were resuspended in 10% trehalose solution to create an iso-osmotic solution for injection and stability in biological fluids was confirmed by scanning electron microscopy for physical integrity and non-clumping (optical activation properties were preserved as well) 72 hrs after incubation in complete mouse serum at 37°C. GNR conjugates were synthesized using classical thiol-maleimide chemistry in three steps as illustrated in Figure 3. The details of the conjugation steps are as follows.

*Step-2 - Activation of antibody* : Anti epidermal growth factor receptor (EGFR) antibody, Cetuximab (2 mg/ml; 152 kDa) is activated by reacting with a heterobifunctional crosslinker, succinimidyl d-[N-maleimidomethyl]cyclohexane-1-carboxylate (SMCC; MW 334.32; arm length 0.83 nm) to expose the maleimide groups for the subsequent conjugation with GNRs containing free sulfhydryl moieties. 2.9 mM of SMCC was prepared in phosphate buffered saline (pH 7.2), containing 2-4 mM EDTA. EDTA is used to chelate the divalent metals, thereby reducing the disulfide formation in the sulfhydryl containing GNRs, for an efficient conjugation. Approximately 20-fold molar excess of 2.9 mM SMCC was added to the antibody solution (1:10 ratio) and incubated for 2 hrs at 4 °C. The maleimide-activated antibody was purified by eluting through a desalting column to remove the unbound cross linker molecules.

*Step-3 - Conjugation of Cetuximab to GNR:* The maleimide-activated Cetuximab (prepared from Step-2) was mixed with the pegylated GNRs with free sulfydryl moieties (prepared from Step-1) at a concentration of ∼ 10 µg/ml. After overnight reaction at 4 °C. the excess maleimide-activated antibody was removed by centrifugation at 10,000 rpm for 20 minutes. The centrifugation step was repeated three time and the pellets were collected and reconstituted in sterile phosphate buffered solution (PBS).

The conjugation efficiency was evaluated by measuring the zeta (ζ) potential of the final conjugates. The CTAB coated GNRs used at the beginning of the conjugation process showed a zeta potential in the range +60 to +80 mV. After the functionalization of GNRs with sulfydryl-PEG the zeta potential decreased to +5 to +10 mV. With the addition of Cetuximab to GNRs, the zeta potential further decreased and reached near neutral values in the range of +4 to -5 mV. These values were consistent among different conjugation batches indicating the efficiency of the surface functionalization and Cetuximab conjugation to the surface of GNRs. Finally the conjugation efficiency was validated by quantifying the ratio of Cetuximab to GNR using micro BCA protein assay. Prior to the protein estimation the optical density (OD) values of pegylated GNRs and C-GNRs at the assay readout wavelength was adjusted to 0.2. PEG-GNR and C-GNRs were subjected to assay protocol and the assay end-product was measured. The endpoint measurements of PEG-GNR samples were subtracted from the C-GNR samples to eliminate the interference of PEG in the estimation of C225 concentration. With the known values of GNR/ml (2x1011 GNR/ml at 1 OD) and the measured concentration of C225 in the C-GNR samples, the ratio of C225 molecules per GNR was estimated as 120 ± 15 C225 molecules/GNR.

### EXAMPLE 3

*In vitro* validation of the conjugates was evaluated in HCT116 cells grown on a chamber slide and incubated for 30 min, 4 and 24 hrs with 200µl of PEG-GNR and C-GNR (1x10¹¹ GNR/ml). Five minutes prior to the end of incubation period 1 µM DAPI (nuclear marker) was added to each well. At the end of each incubation period the cells were fixed in 2% paraformaldehyde and visualized by dark field microscopy. As noted in Figure 4 greater accumulation and internalization of conjugated GNRs was observed at 24 hrs than at earlier time points. When compared to the PEG-GNRs, C-GNRs displayed excellent cell-surface binding (Figure 4), which was confirmed by the blocking experiment with cetuximab pre-treatment.

### EXAMPLE 4

This functionalization chemistry is useful for any gold surface or other targeting molecules. For example, folic acid (FA)has been used as a targeting molecule. Briefly, SH-PEG5K-NH2 was added to a solution of gold nanorods to bring the SH-PEG5K-NH2 concentration to ∼0.3 mM. After stirring overnight, excess SH-PEG5K-NH2 was removed via diafiltration into dI water. The terminal NH2 group on the PEG would then be used to crosslink to the folic acid (FA). The cross linker 1-Ethyl-3-[3-dimethylaminopropyl]carbodiimide Hydrochloride (EDC) was added to a solution containing equimolar amounts of free FA and the SH-PEG-NH2 functionalized nanorods. The EDC crosslinks the PEG and the folic acid by activating a carboxylic acid group on the folic acid which would subsequently bind covalently to the amine on the PEG forming a stable amide bond. However, we noted a near immediate irreversible aggregation of the PEGylated particles upon addition of FA to the solution.

FA-conjugated nanorods were also prepared by first conjugation the SH-PEG5K-NH2 with FA prior to the addition to the nanorods. In this reaction, we added EDC to a solution of bifunctional PEG and a molar excess of FA. Excess ECD and FA were then removed by dialysis. The SH-PEG5K-FA was added to a nanorod solution to bring the PEG concentration to ∼ 0.3 mM. Using this method, no aggregation of the particles was observed. After an overnight incubation, excess SH-PEG5K-FA was removed by diafiltration into phosphate buffered saline.

### Reference example 5

### FABRICATION AND CONJUGATION OF TARGETED GOLD NANOSHELLS

The conjugation chemistry previously described may be adapted to other targeting molecules as well other particles. Gold nanoshells are another class of NPs with a high-Z content. The methods for manufacturing of these particles are described in US Patents 6,344,272, 6,685,986 and 7,371,457. Other methods of production include "hollow" nanoshells manufactured by reduction of gold onto a more reactive core (Melancon, et al. 2008; Lu, et al. 2009). The gold surface of NPs allows the use of a bifunctional PEG to attach the targeting molecule.

Nanoshells were synthesized as previously described. Nanoshell formation was assessed using an ultraviolet-visible (UVVIS) spectrophotometer (U-0080D, Hitachi) and Zetasizer (Nano-ZS, Malvern Instruments, Malvern, UK). The Integrin αᵥβ₃-targeting peptide cyclo-(RGDfK) (molecular weight [MW] = 617.71) and control peptide cyclo-(Arg-Ala-Asp-D-Phe-Lys) (cyclo-(RADfK), MW = 603.68) were purchased from Peptides International (Louisville, KY, USA). Integrin αvβ3 antagonist IAC (MW = 622.14) was provided by Dr Narasimhan Danthi from the Molecular Imaging Laboratory at National Institutes of Health. Bifunctional ortho-pyridyldisulfide-polyethylene glycol 2000-N-hydroxysuccinimide ester (OPSS-PEG2k-NHS) was purchased from Nektar (Huntsville, AL, USA). Chelating agent S-2-(4-aminobenzyl)-1,4,7,10-tetraazacyclododecane tetraacetic acid (DOTA) was purchased from Macrocyclics (Dallas, TX. USA). The free amine groups of RGDfK, RADfK, IAC, and DOTA were conjugated to OPSS-PEG-NHS by mixing 1:1 molar ratios overnight at pH 7.4 at room temperature (RT). The resulting OPSS-PEG conjugates were then mixed with the nanoshell solution (in 10 mM phosphate buffer, pH7) at 10,000:1 molar ratio overnight at RT on a shaker, allowing the OPSS group to conjugate to the gold surface of the particles. The mixture was centrifuged, and the supernatant with unconjugated OPSS-PEG was removed. The pellets of NS-peptide, NS-IAC, or NS-DOTA were resuspended in phosphate buffer and analyzed by a spectrophotometer and Zetasizer to determine the nanoshell concentration and size, respectively, for further conjugation. To prepare nanoshells with both RGDfK and DOTA on the surface, OPSS-PEG-RGDfK and OPSS-PEG-DOTA were mixed with nanoshells at a 5000:1 molar ratio followed by the incubation and separation steps.

### EXAMPLE 6

In one example, the [Nle4, D-Phe7]-α-Melanocyte Stimulating Hormone (NDP-MSH) was conjugated to the NP. The NDP-MSH peptide was first conjugated to a -5000 MW heterobifucntional PEG. The bifunctional PEG has a thiol (-SH) group on one end through which the PEG will be attached to the particle. The other end of the PEG has an activated NHS-ester which will bind to the terminal amine on the NDP-MSH peptide. After the NDP-MSH was reacted with the bifunctional PEG for 2 hours it was added to the nanoparticle solution. As an experimental control, a 5000 MW PEG was added to a second solution of nanoparticles. Both solution were allowed to react overnight at 2-8° C. Excess PEG not attached to the nanoparticles was removed by centrifugation of the particles and resuspension in deionized water.

Conjugation of the NDP-MSH was confirmed using an in vitro binding experiment comparing the binding density of NDP-MSH conjugated NP to the NP coated with PEG to melanocytes. Cells were plated onto microscope slides so that binding could be observed by microscope. The slides were then exposed to either the NDP-MSH NP or the PEG NP. The cells were incubated with the particles for 1 hr at 37 °C. After removal of any unbound particles, the slides were imaged in dark field to determine the amounts of particles bound to the cells. Microscope images taken from slides, with two microscope images overlaid, a phase contrast image, which localizes the cells, and a darkfield image which localized the particles. By overlaying the two images the presence of the particles were observed indicating affinity of the NDP-MSH NP for the cells.

Similarly, folic acid and the HER-2 antibody were conjugated to nanoshells.

### EXAMPLE 7

### GNRS INCREASE BIOLOGICAL EFFECTIVENESS OF RADIATION IN VIVO AND IN VITRO

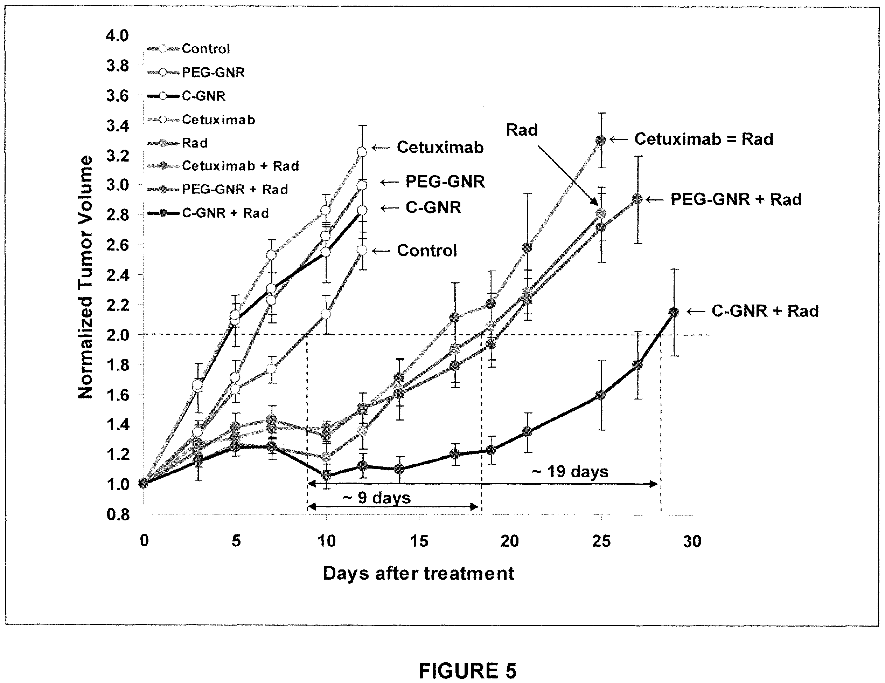

In vivo radiosensitization was evaluated in a subcutaneous HCT116 colorectal cancer model that is known to be resistant to cetuximab (K-Ras mutant). PEG-GNRs or C-GNRs (∼2x10¹¹ GNRs each) were injected via the tail vein 24 hrs prior to a tumor-localized dose of radiation (10Gy) from a 250kVp orthovoltage unit. The time it takes for tumor volume to double (measure of local radiosensitization) was noted for each of multiple groups (see Figure 5). Also note that HCT116 is resistant to cetuximab treatment due to a K-ras mutation - therefore, the radiation sensitization was not due to the cetuximab,. This has been confirmed experimentally in a parallel in vivo tumor regrowth delay experiment as well. Whereas the combination of PEG-GNR with radiation did not increase time to tumor volume doubling, the combination of C-GNR with radiation nearly doubled the time to tumor volume doubling (19 days vs. 10 days, p<0.001). The time it takes for a tumor to double in volume after conjugated GNR + radiation was nearly 1.9 times that for tumors after unconjugated GNR + radiation.

Inductively coupled plasma - mass spectrometry (ICP-MS) analysis of the biodistribution of GNRs and conjugated GNRs 24 hrs after intravenous injection in nude mice demonstrated slightly higher accumulation of conjugated GNRs within tumors than unconjugated GNRs. Individual organs were harvested 24 hrs after injection of ∼2x10¹¹ GNRs or C-GNRs via the tail vein to mice bearing HCT116 xenografts. By injecting less than 0.0002% by mass of the body weight, we showed tumor gold accumulation of much less than 0.00007% by mass of the tumor, i.e., substantially lower than the gold accumulation typically believed necessary for dose enhancement. See Figure 6. C-GNRs accumulated more within tumors than GNRs.

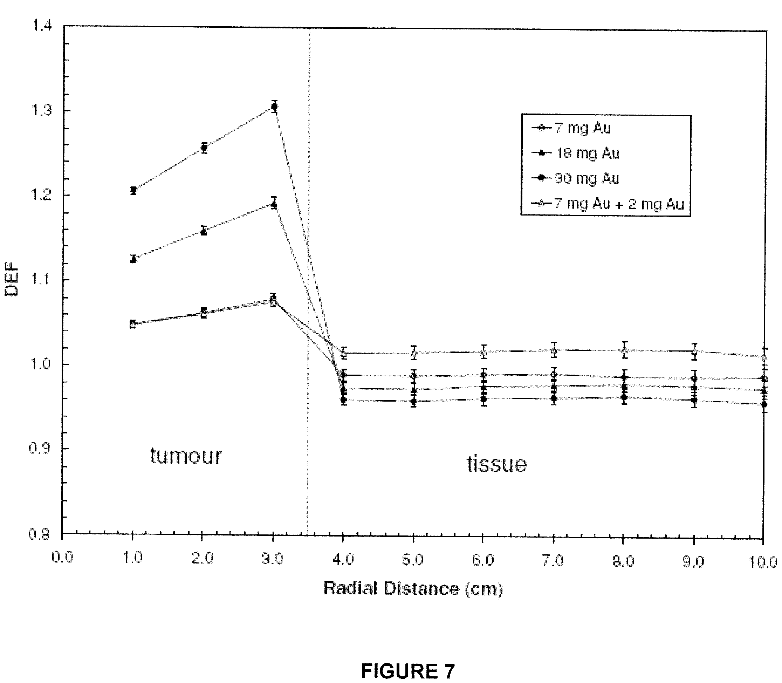

Figure 7 illustrates estimates (Cho, 2005) of the extent of radiation dose enhancement expected if the gold particles are evenly distributed throughout the tumor. Although the measured gold content in tumors is higher with conjugation, this would be insufficient to cause any realistic dose enhancement based on such modeling, but dose enhancement was observed in vivo as illustrated in Figure 5.

In this example, the total injected dose of GNR in mice was less than 0.05% by mass of the body weight. Additionally, the dose accumulation in the tumor was substantially less than 0.05% by mass of the tumor mass (see Figure 6 and Table 1). The dose enhancement was observed with the cetuximab-targeted GNRs plus radiation, but not from the untargeted GNRs plus radiation. The dose enhancement observed from these low doses of targeted GNRs may be attributed to the greater retention of these particles in the tumor and the closer proximity to the nucleus, increasing the radiation effect on the tumor cell. Higher doses of particles, as well as serial doses, with single dose or multiple fraction irradiations would result in similar dose enhancement. Additionally, the voltage of radiation may be selected to allow proper irradiation of the tumor at depth consistent with the high-Z material mass present in the tumor.

**Table 1**

| | | **Gold Mass Injected (ug)** | **% Gold Injected by Approx. Body Weight** | **Mass Gold in Tumor (ug)** | **Tumor Mass (g)** | **Gold in Tumor as % Tumor Mass** |
|---|---|---|---|---|---|---|
| PEGylated Rods | Animal # 151 | 28.537 | 0.00011% | 0.126 | 1.277 | 0.000010% |
| PEGylated Rods | Animal # 152 | 28.537 | 0.00011% | 0.126 | 1.236 | 0.000010% |
| PEGylated Rods | Animal # 153 | 28.537 | 0.00011% | 0.117 | 1.135 | 0.000010% |
| PEGylated Rods | Animal # 154 | 28.537 | 0.00011% | 0.155 | 0.914 | 0,000017% |
| Targeted Rods | Animal # 155 | 28.537 | 0.00011% | 0.480 | 0.915 | 0.000052% |
| Targeted Rods | Animal # 156 | 28.537 | 0.00011% | 0.328 | 1.013 | 0.000032% |
| Targeted Rods | Animal # 157 | 28.537 | 0.00011% | 0.414 | 1.171 | 0.000035% |
| Targeted Rods | Animal # 158 | 28.537 | 0.00011% | 0.359 | 0.806 | 0.000045% |

To understand the mechanism of this dramatic radiosensitization, in vitro clonogenic survival assays were performed where significant enhancement (p<0.001) of HCT116 radioresponse was observed with a solution of 100 ul of 0.2% GNRs by weight applied to cells (Figure 8). A hundred-fold lower concentration of C-GNRs (0.002% by weight of the solution added) had nearly similar radiosensitization potential as the 0.2% GNRs. Another significant difference between these two experiments is the presence of GNRs in the cell culture media during the radiation dose delivery. With 0.2 % GNRs the cells were irradiated with the entire concentration of GNRs present in the media. Whereas, in the case of 0.002 % C-GNRs the media containing C-GNRs was aspirated, fresh media was added and the cells were irradiated in the presence of C-GNRs that were bound to the cell surface receptors. These clonogenic survival of HCT116 cells exposed to unconjugated and conjugated GNRs suggest that a dose-enhancement of 10% can be achieved at 30 mins and 15% at 24 hrs following a single dose of kilovolt (250 kVp) radiation. Data is typically represented as a log-linear curve with the percentage of surviving colonies of cancer cells represented on a log scale. Consequently, the separation of the curves noted in these figures is substantial and clinically meaningful, particularly since this is a modeling of a single radiation dose. Extrapolating these data to typical multiple-fraction radiation treatments for cancer (typically around 25 treatments), the single-fraction dose enhancement factor of 1.15 would represent a (1.15)²⁵ cumulative dose enhancement in an idealized situation.

Extrapolating these data to typical multiple-fraction external beam radiation treatments for rectal cancer (typically 28 treatments), the single-fraction dose enhancement factor of 1.2 (0.002% C-GNRs) to 1.3 (0.2% GNRs) would represent a (1.2)²⁸ to (1.3)²⁸ cumulative dose enhancement in an idealized situation.

### EXAMPLE 8

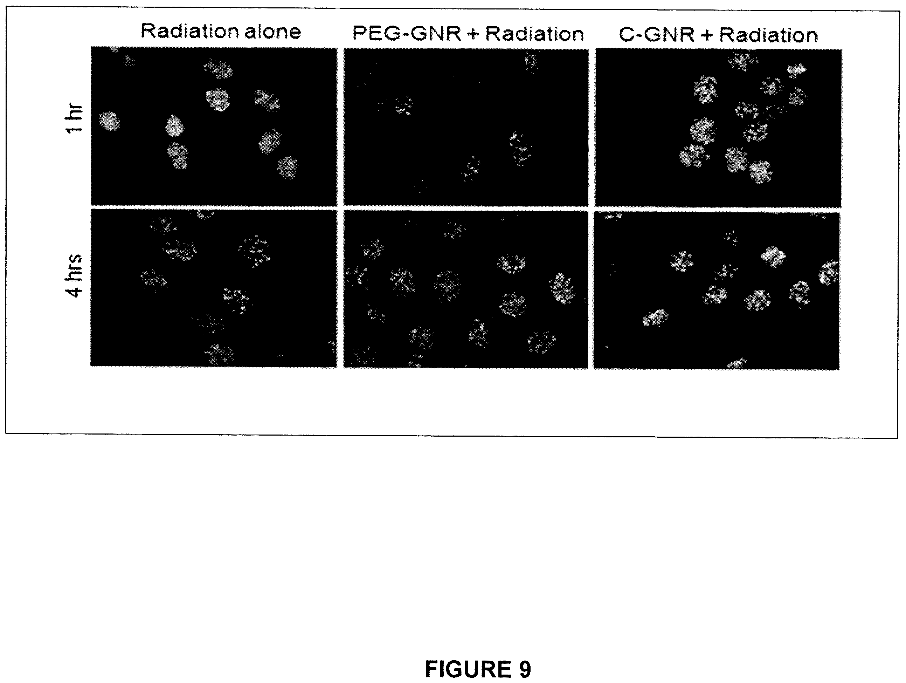

To understand the mechanism of radiosensitization further, immunofluorescence imaging was performed on cells in chamber slides exposed to a single dose of 2Gy in the presence and absence of PEG-GNRs and C-GNRs (1x10¹¹ GNR/ml) and the number of phosphorylated histone 2AX (γ-H2AX) foci present within nuclei was estimated at different time points. Histone 2 AX is recruited to sites of DNA strand breaks and activated (gamma H2AX). The presence of these foci serves as a surrogate for un-repaired DNA double strand breaks typically seen immediately after radiation - higher intrinsic biological effectiveness (either due to higher physical dose seen at the level of the cellular nucleus/DNA or slower physiological repair of DNA strand breaks would cause these γ-H2AX foci to disappear more slowly). In these experiments, substantially more γ-H2AX foci were observed at 1 and 4 hrs after radiation in the presence of C-GNRs than PEG-GNRs (Figure 9).

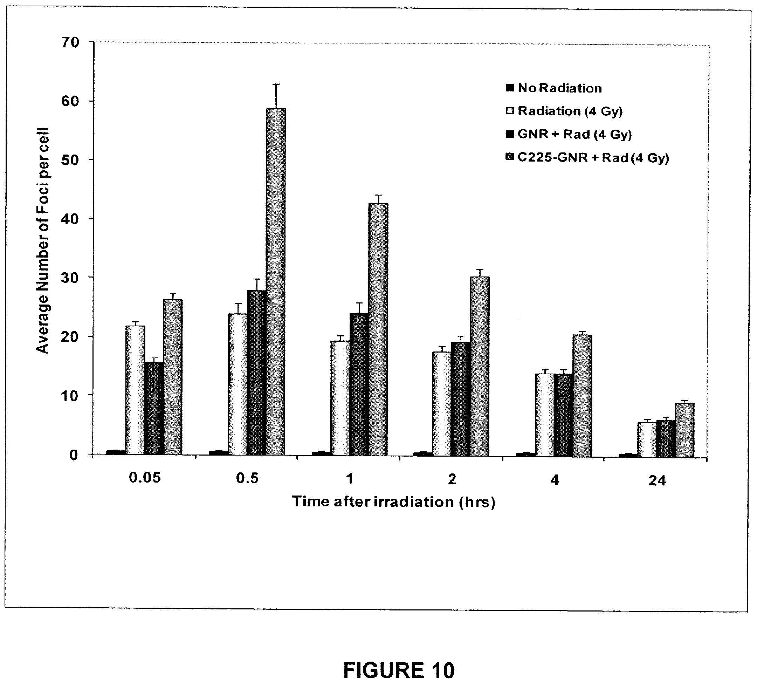

The γ-H2AX foci in each image were quantified and the results are illustrated as a bar chart in Figure 10, including a timepoint 24 hours after radiation.

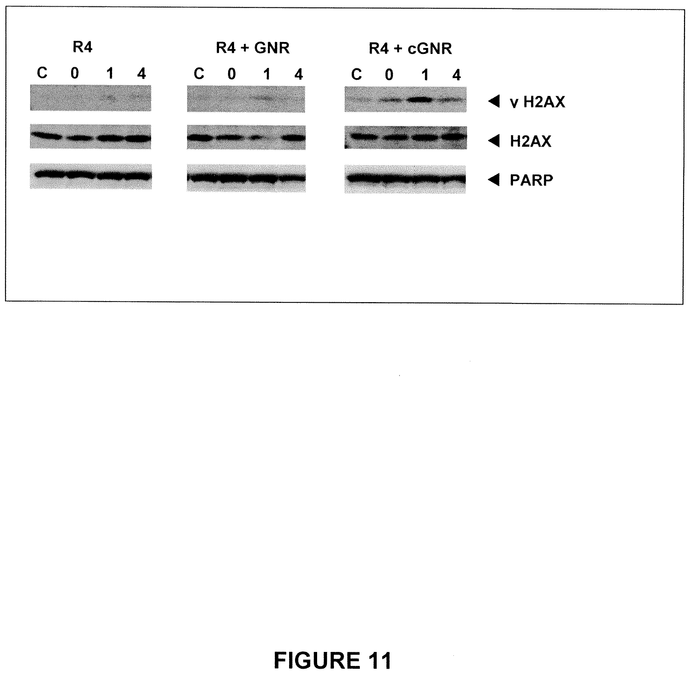

A similar set of experiments were performed and the cytoplasmic and nuclear extracts were collected for western blot analysis. A Western blot analysis was performed to evaluate the downstream DNA damage signaling proteins and γ-H2AX. The western blot analysis of γ-H2AX demonstrated the similar trend as observed with immunofluorescence staining and the results are included in Figure 11.

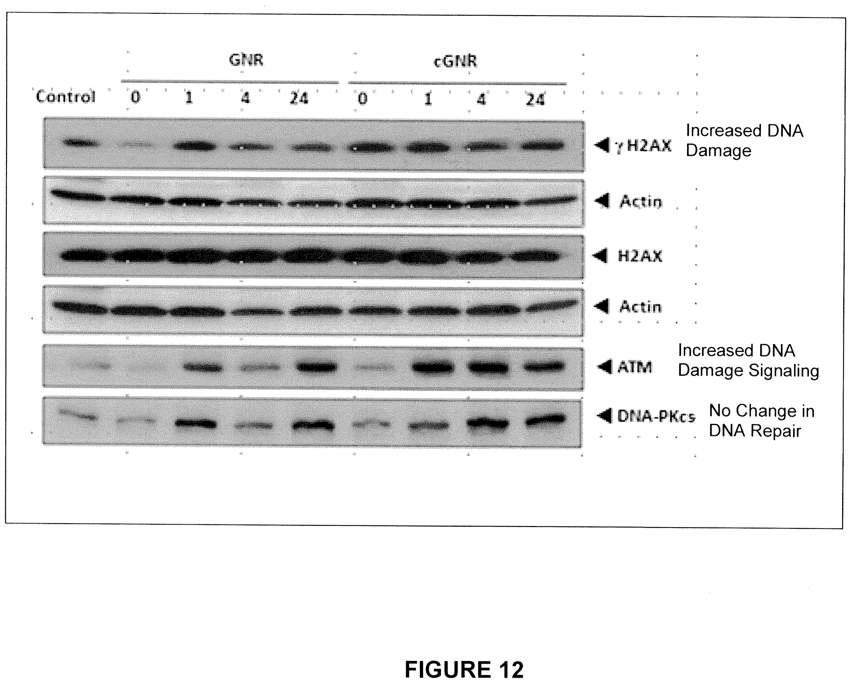

A Western blot analysis of gamma H2AX and increased DNA damage signaling downstream was performed in the extracted HCT116 tumors. See Figure 12. No difference was noted in DNA repair proteins. This increased DNA damage was also evident in tumor tissues 24 hrs after radiation. The damage was more evident in the cetuximab targeted GNRs.

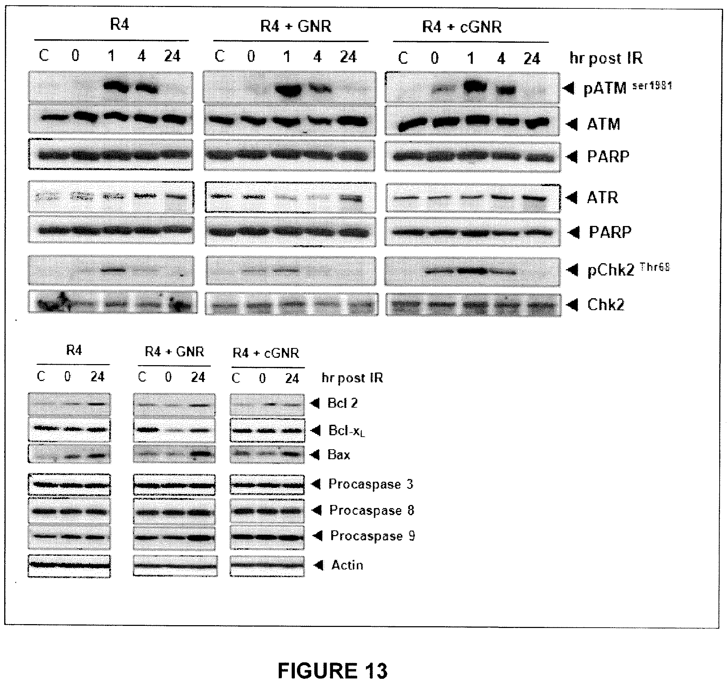

A Western blot analysis was also performed of nuclear and cytoplasmic extracts *in vitro* of HTC116 cells after GNR and cGNR plus radiation. See Figure 13. The Western blot analysis of nuclear extracts confirmed the increase in DNA damage signaling downstream of the strand breaks (pATM, ATR and chk2 being the sensors and signaling molecules that are downstream of gamma H2AX). The Western blot analysis of cytoplasmic extracts, compared to the radiation + GNR group, indicated an increase in bcl2/bax ratio without a change in caspases in the radiation + C-GNR group, which indicates a change in mitochondrial membrane potential.

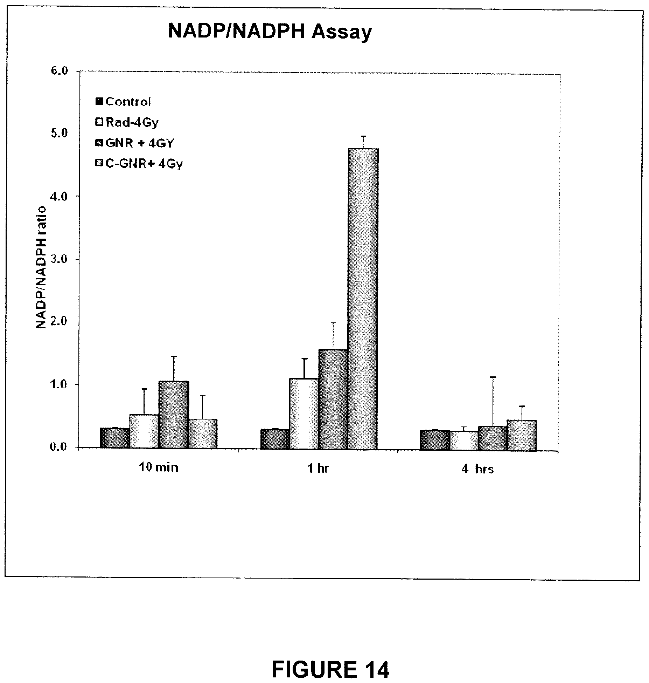

The mitochondrial redox potential was measured after radiation in the presence of conjugated GNRs compared to unconjugated GNRs. See Figure 14. Under physiological conditions, the equilibrium between NADP and NADPH favors the formation of NADPH but during oxidative stress, there is a shift in this equilibrium towards greater formation of NADP. The increased NADP/NADPH ratio in the C-GNR cells indicates increased oxidative stress. In brief, cells grown in multiple 60mm culture plates were incubated with 2ml of 0.5 OD GNR in culture media (1x10¹¹ GNR/ml) for 24 hrs. At the end of incubation time the media containing GNRs were aspirated and irradiated 4 Gy (using 250 kVp X-ray) in the presence of fresh culture media. The cells were collected immediately, 1hr, 4hrs after the radiation and processed for NADP⁺/NADPH assay. The assay end product was measured using spectrophotometer and the amount of NADP⁺ and NADPH was estimated using the calibration graph generated from the known standard samples.

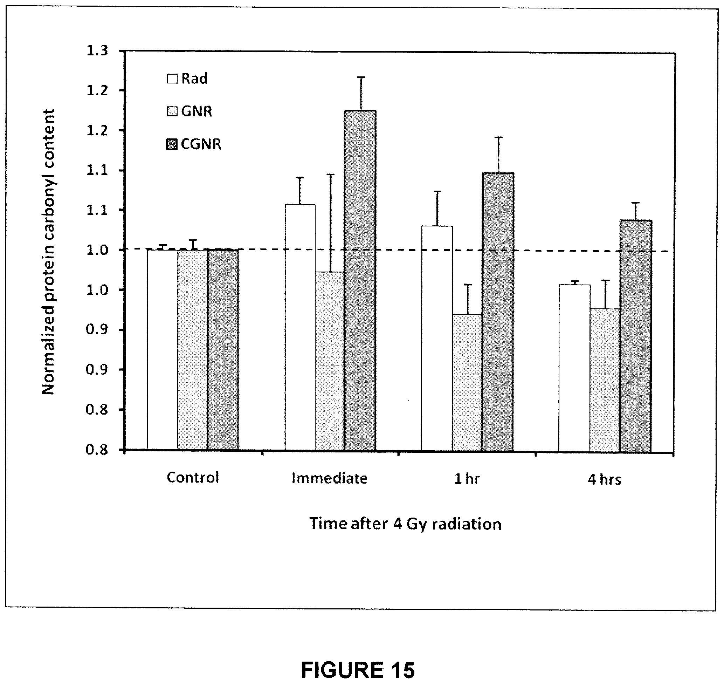

Irradiation in the presence of C-GNRs resulted in greater oxidative modification of proteins globally than irradiation in the presence of unconjugated GNRs. See Figure 15. The protein carbonyl assay measures the covalent modification of proteins caused directly by reactive oxygen species (ROS) or by by-products of oxidative stress. This serves as a stable marker of oxidative modification of proteins detectable immediately in response to ROS-induced oxidative stress. The experimental procedures, GNR concentration, incubation time, irradiation and extraction time are exactly the same as done for NADP⁺/NADPH. The cell lysates were prepared as required for the protein carbonyl assay and the end product of the assay was measured using spectrophotometer and protein carbonyl content was estimated from the calibration graph generated from the known standard samples.

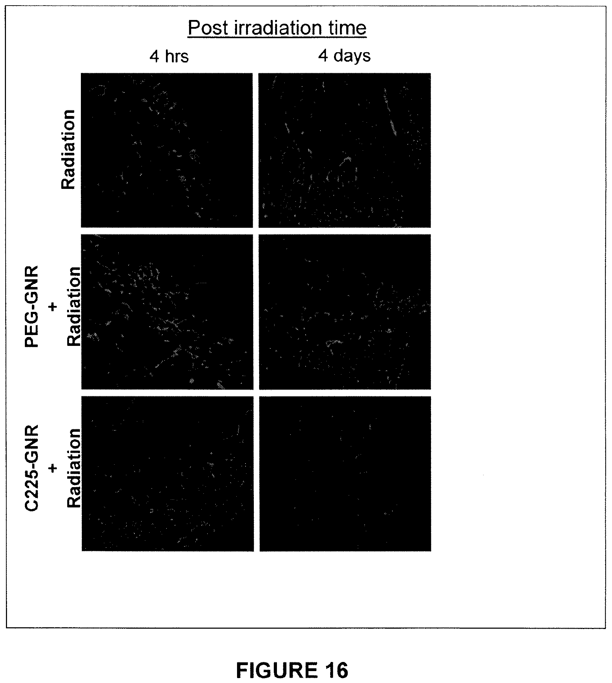

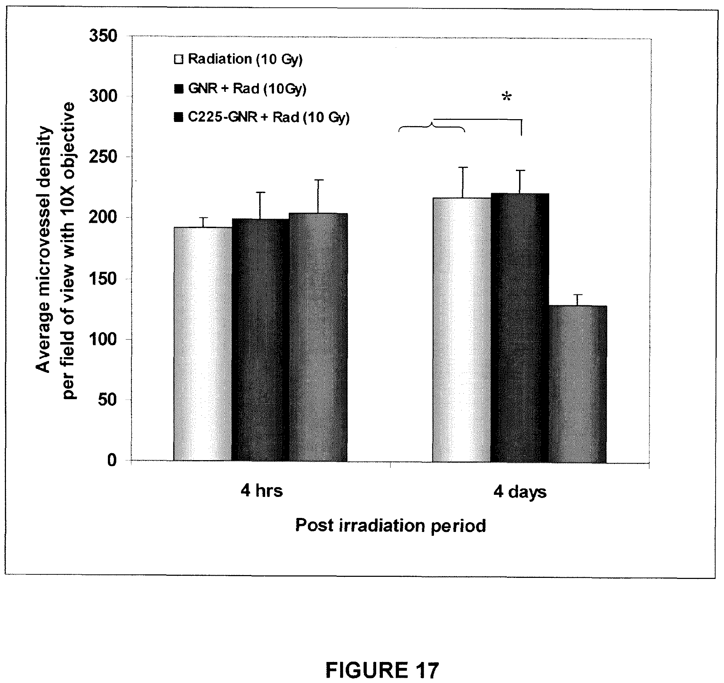

Substantial reduction in markers of tumor vascularity was observed when tumors were radiated (10 Gy using 230 kVp X-rays) after treatment with C-GNRs (∼2.1x10¹¹ GNR; 5.2 OD 200 µL injection via tail vein) compared to PEG-GNRs (∼2.1x10¹¹ GNR; 5.2 OD 200 µL injection via tail vein). To access the immediate and late effects of C-GNR mediated radiosensitization on the tumor vasculature the animals were euthanized at 4hrs and 4 days after the radiation and the tumor tissue was collected and snap frozen with optical cutting media in liquid nitrogen. The frozen tissues were sectioned and stained for vascular endothelial markers CD31. A significant reduction in the vascular staining was noted 4 days after radiation in the C-GNR treated tumors. See Figure 16. The average microvessel density per field of view is illustrated in Figure 17. This indicates that conjugated GNRs exert their radiosensitization effects via two distinct mechanisms - first, their greater internalization into cancer cells results in greater intracellular concentration for greater oxidative stress (mitochondrial membrane potential destabilization) and the greater proximity to DNA results in more pronounced and prolonged DNA damage (that is both sensed and propagated downstream via signaling molecules). Second, their greater accumulation in the perivascular space results in greater damage to vascular endothelial cells and decrease in microvessel density.

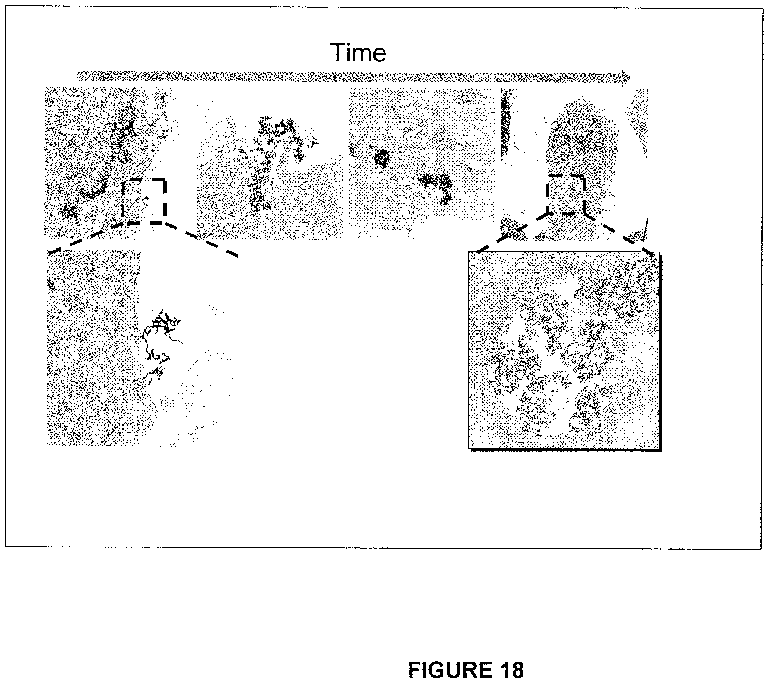

Transmission electron microscopic (TEM) images document the progressive internalization of conjugated (but not unconjugated) GNRs into cellular cytoplasm after cell-membrane binding. HCT116 cells growing on coverslips were treated with unconjugated (not shown - no internalization observed) and conjugated GNRs and fixed at different time points thereafter for TEM imaging. See Figure 18.

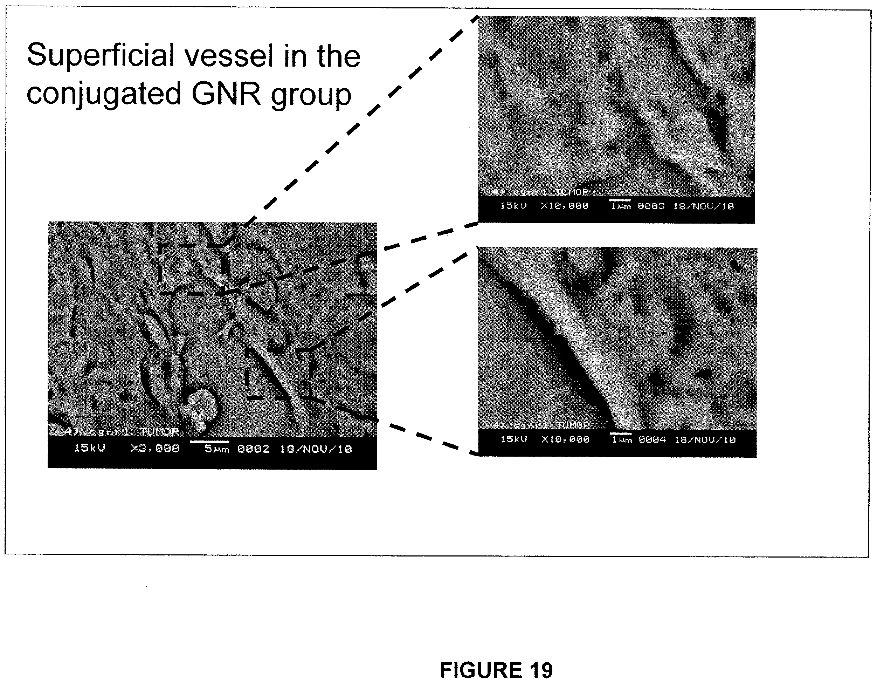

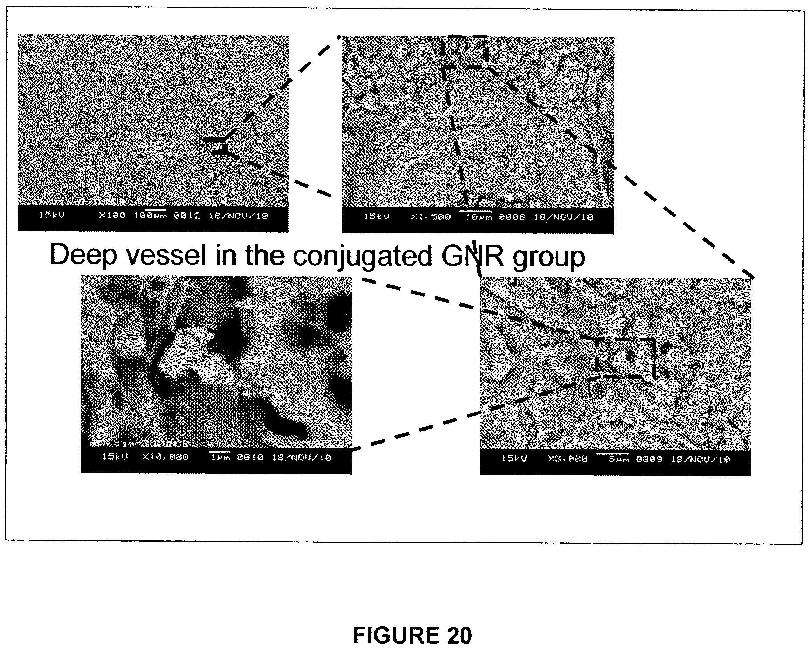

TEM analysis of tumor tissue distribution confirmed the perivascular accumulation of both unconjugated and conjugated GNRs. Perivascular accumulation was more prominent in the case of conjugated than unconjugated GNRs. See Figures 19 and 20.

### EXAMPLE 9

### RADIATION DOSE ENHANCEMENT OF OTHER TUMOR TYPES

The EPR effect has been observed in numerous tumor types that would allow NP accumulation. These tumor types also have cell surface receptors that would enable tumor targeting. These cancers include colorectal, brain, lung, pancreatic, renal, breast, ovarian, uterine, endometrial, squamous cell, melanoma, and prostate, among others. Additionally, this dose enhancement method would be useful for metastatic disease in these and other cancer types.

### REFERENCES CITED

The following references provide exemplary procedural or other details supplementary to those set forth herein.

### U.S. PATENT DOCUMENTS

US Patent No. 6,955,639, dated October 18, 2005, with Hainfeld, et al., listed as inventors;
US Patent No. 7,194,063, dated March 20, 2007, with Dilmanian, et al., listed as inventors;
US Patent No. 7,367,934, dated May 6, 2008, with Hainfeld, et al., listed as inventors;
US Patent No. 7.530,940, dated May 12, 2009, with Hainfeld, et al., listed as inventors;
US Patent No. 6,344,272, dated February 5, 2002, with Oldenburg, et al., listed as inventors;
US Patent No. 6,685,986, dated February 3, 2004, with Oldenburg, et al., listed as inventors;
US Patent No. 7,371.457, dated May 13, 2008, with Oldenburg, et al., listed as inventors; and
US Patent Publication No. 2009/0186060, with Hainfeld, et al. listed as inventors.
US Patent Publication No. 2011/00052672, with Krishnan, et al. listed as inventors.

### NON-PATENT DOCUMENTS

Baselga, J. (2006). "Targeting tyrosine kinases in cancer: the second wave." Science 312(5777): 1175-8.
Beddar, A. S., S. Krishnan, et al. (2006). "The optimization of dose delivery for intraoperative high-dose-rate radiation therapy using curved HAM applicators." Radiother Oncol 78(2): 207-12.
Blume-Jensen, P. and T. Hunter (2001). "Oncogenic kinase signalling." Nature 411(6835): 355-65.
Bonner, J. A., P. M. Harari, et al. (2006). "Radiotherapy plus cetuximab for squamous-cell carcinoma of the head and neck." N Engl J Med 354(6): 567-78.
Caliceti, P. and F. M. Veronese (2003). "Pharmacokinetic and biodistribution properties of poly(ethylene glycol)-protein conjugates." Adv Drug Deliv Rev 55(10): 1261-77.
Cho, S., O. Vassiliev, et al. (2007). Microscopic estimation of tumor dose enhancement during gold nanoparticle-aided radiation therapy (GNRT) using diagnostic energy range x-rays. (abstr.). Med Phys. 34: 2468.
Cho, S. H. (2005). "Estimation of tumour dose enhancement due to gold nanoparticles during typical radiation treatments: a preliminary Monte Carlo study." Phys Med Biol 50(15): N163-73.
Cho, S. H., B. L. Jones, et al. (2009). "The dosimetric feasibility of gold nanoparticle-aided radiation therapy (GNRT) via brachytherapy using low-energy gamma-/x-ray sources." Phys Med Biol 54(16): 4889-905.
Choi, H. S., W. Liu, et al. (2007). "Renal clearance of quantum dots." Nat Biotechnol 25(10): 1165-70.
Cunningham, D., Y. Humblet, et al. (2004). "Cetuximab monotherapy and cetuximab plus irinotecan in irinotecan-refractory metastatic colorectal cancer." N Engl J Med 351(4): 337-45.
Dvorak, H. F., J. A. Nagy, et al. (1988). "Identification and characterization of the blood vessels of solid tumors that are leaky to circulating macromolecules." Am J Pathol 133(1): 95-109.
Goldberg, R. M. (2005). "Cetuximab." Nat Rev Drug Discov Suppl: S10-1.
Haddock, M. G., L. L. Gunderson, et al. (2001). "Intraoperative irradiation for locally recurrent colorectal cancer in previously irradiated patients." Int J Radiat Oncol Biol Phys 49(5): 1267-74.
Hainfeld, J. F., D. N. Slatkin, et al. (2004). "The use of gold nanoparticles to enhance radiotherapy in mice." Phys Med Biol 49(18): N309-15.
Hainfeld, J. F., D. N. Slatkin, et al. (2004). "The use of gold nanoparticles to enhance radiotherapy in mice." Physics in medicine and biology 49(18): N309-15.
Hauck, T. S., A. A. Ghazani, et al. (2008). "Assessing the effect of surface chemistry on gold nanorod uptake, toxicity, and gene expression in mammalian cells." Small 4(1): 153-9.
Huang, X., P. K. Jain, et al. (2007). "Gold nanoparticles: interesting optical properties and recent applications in cancer diagnostics and therapy." Nanomedicine (Lond) 2(5): 681-93.
Huang, X., X. Peng, et al. "A reexamination of active and passive tumor targeting by using rod-shaped gold nanocrystals and covalently conjugated peptide ligands." ACS Nano 4(10): 5887-96.
Huff, T. B., M. N. Hansen, et al. (2007). "Controlling the cellular uptake of gold nanorods." Langmuir 23(4): 1596-9.
Jana, N. R., L. Gearheart, et al. (2001). "Wet chemical synthesis of high aspect ratio cylindrical gold nanorods." Journal of Physical Chemistry B 105: 4065-4067.
Karapetis, C. S., S. Khambata-Ford, et al. (2008). "K-ras mutations and benefit from cetuximab in advanced colorectal cancer." N Engl J Med 359(17): 1757-65.
Krishnan, S., N. A. Janjan, et al. (2006). "Phase II study of capecitabine (Xeloda) and concomitant boost radiotherapy in patients with locally advanced rectal cancer." Int J Radiat Oncol Biol Phys 66(3): 762-71.
Lee, S. Y., M. Ferrari, et al. (2009). "Shaping nano-/micro-particles for enhanced vascular interaction in laminar flows." Nanotechnology 20(49): 495101.
Lu, W., C. Xiong, et al. (2009). "Targeted photothermal ablation of murine melanomas with melanocyte-stimulating hormone analog-conjugated hollow gold nanospheres." Clin Cancer Res 15(3): 876-86.
Maeda, H., J. Fang, et al. (2003). "Vascular permeability enhancement in solid tumor: various factors, mechanisms involved and its implications." Int Immunopharmacol 3(3): 319-28.
Manning, H. C., N. B. Merchant, et al. (2008). "Molecular imaging of therapeutic response to epidermal growth factor receptor blockade in colorectal cancer." Clin Cancer Res 14(22): 7413-22.
Melancon, M. P., W. Lu, et al. (2008). "In vitro and in vivo targeting of hollow gold nanoshells directed at epidermal growth factor receptor for photothermal ablation therapy." Mol Cancer Ther 7(6): 1730-9.
Mitragotri, S. and J. Lahann (2009). "Physical approaches to biomaterial design." Nat Mater 8(1): 15-23.
Papillon, J. (1990). "Present status of radiation therapy in the conservative management of rectal cancer." Radiother Oncol 17(4): 275-83.
Pissuwan, D., T. Niidome, et al. (2009). "The forthcoming applications of gold nanoparticles in drug and gene delivery systems." J Control Release.
Rodel, C., D. Arnold, et al. (2008). "Phase I-II trial of cetuximab, capecitabine, oxaliplatin, and radiotherapy as preoperative treatment in rectal cancer." Int J Radiat Oncol Biol Phys 70(4): 1081-6.
Saltz, L. B., N. J. Meropol, et al. (2004). "Phase II trial of cetuximab in patients with refractory colorectal cancer that expresses the epidermal growth factor receptor." J Clin Oncol 22(7): 1201-8.
Unezaki, S., K. Maruyama, et al. (1996). "Direct measurement of the extravasation of polyethyleneglycol-coated liposomes into solid tumor tissue by in vivo fluorescence microscopy." Int. J. Pharm 144: 11-17.
Van Cutsem, E., C. H. Kohne, et al. (2009). "Cetuximab and chemotherapy as initial treatment for metastatic colorectal cancer." N Engl J Med 360(14): 1408-17.
Zharov, V. P., J. W. Kim, et al. (2005). "Self-assembling nanoclusters in living systems: application for integrated photothermal nanodiagnostics and nanotherapy." Nanomedicine 1(4): 326-45.

## Claims

1. High-Z particles for use in a method for enhancing the effects of radiation directed to a tissue or a population of cells, wherein the high -Z particles are gold nanorods and have a surface coating of polyethylene glycol (PEG);
the high-Z particles comprise a targeting molecule with an affinity for a targeted tissue or a targeted population of cells;
wherein the high-Z particles are administered to an animal and the targeted tissue or targeted population of cells is subsequently irradiated with ionizing radiation;
wherein the high-Z particles are administered to the animal in an amount sufficient to achieve a concentration in the targeted tissue or the targeted population of cells of less than 0.05% metal by weight.

2. The particles for use according to claim 1, wherein the targeting molecule results in the internalization of the high-Z particles upon binding with the targeted tissue or the targeted population of cells.

3. The particles for use according to claim 1, wherein the ionizing radiation is delivered in fractions over a period of time.

4. The particles for use according to claim 1, wherein the high-Z particles are administered to the animal one or more times.

5. The particles for use according to claim 1, wherein the targeted tissue or targeted population of cells is irradiated within the animal.

6. The particles for use according to claim 1, wherein the targeted tissue or targeted population of cells comprises cancer cells.

7. The particles for use according to claim 6, wherein the cancer cells are selected from the group consisting of: primary or metastatic colorectal cancer cells, brain cancer cells, lung cancer cells, pancreatic cancer cells, renal cancer cells, breast cancer cells, ovarian cancer cells, uterine cancer cells, endometrial cancer cells, squamous cancer cells, melanoma cancer cells, and prostate cancer cells.

8. The particles for use according to claim 1, wherein the tissue or population of cells is irradiated extracorporeally.

9. The particles for use according to claim 6, wherein the targeted tissue or targeted population of cells is circulating tumor cells or blood cells.

10. The particles for use according to claim 1, wherein the high-Z material is a particle with an average diameter of between approximately 8 nm and 200 nm.

11. The particles for use according to claim 1, wherein the targeting molecule has an affinity for a receptor expressed in cancer cells.

12. The particles for use according to claim 1, wherein the targeting molecule has an affinity for a target selected from the group consisting of: human epidermal growth factor receptor 2, human epidermal growth factor receptor 3, human epidermal growth factor receptor 4, vascular endothelial growth factor receptor, folic acid receptor, melanocyte stimulating hormone receptor, integrin avb3, insulin-like growth factor receptor, hepatocyte growth factor receptor, and basic fibroblast growth factor receptor.

13. The particles for use according to claim 1, wherein the targeting molecule is selected from the group consisting of: an antibody to the target receptor; a peptide, protein, aptamer, oligomer, or small molecule with affinity for the target receptor; and combinations thereof.

14. The particles for use according to claim 1, wherein the targeting molecule is selected from the group consisting of: cetuximab, herceptin, folic acid, melanocyte stimulating hormone, cyclic-RGD, and an analogue to cyclic-RGD.

15. The particles for use according to claim 1, wherein the ionizing radiation is delivered continuously through an implant in or near the tumor of a radiation-emitting substance.

## Patentansprüche

1. High-Z-Partikel zur Verwendung in einem Verfahren zur Verbesserung der Wirkungen von Bestrahlung, die auf ein Zellgewebe oder eine Zellpopulation gerichtet ist, wobei die High-Z-Partikel Goldnanostäbchen (gold nanorods) sind und eine Oberflächenbeschichtung von Polyethylenglycol (PEG) aufweisen; die High-Z-Partikel ein Zielmolekül mit einer Affinität für ein Zielgewebe oder eine Zielzellpopulation umfassen;
wobei die High-Z-Partikel an ein Tier verabreicht werden und das Zielgewebe oder die Zielzellpopulation anschließend mit ionisierender Strahlung bestrahlt wird;
wobei die High-Z-Partikel dem Tier in einer Menge verabreicht werden, die ausreichend ist, um in dem Zielgewebe oder der Zielzellpopulation eine Konzentration von weniger als 0,05 Gew.-% Metall zu erreichen.

2. Partikel zur Verwendung nach Anspruch 1, wobei das Zielmolekül zur Internalisierung der High-Z-Partikel nach Bindung an das Zielgewebe oder die Zielzellpopulation führt.

3. Partikel zur Verwendung nach Anspruch 1, wobei die ionisierende Strahlung in Einzeldosen (Fraktionen) über einen Zeitraum erfolgt.

4. Partikel zur Verwendung nach Anspruch 1, wobei die High-Z-Partikel dem Tier einmal oder mehrere Male verabreicht werden.

5. Partikel zur Verwendung nach Anspruch 1, wobei das Zielgewebe oder die Zielzellpopulation innerhalb des Tiers bestrahlt wird.

6. Partikel zur Verwendung nach Anspruch 1, wobei das Zielgewebe oder die Zielzellpopulation Krebszellen umfasst.

7. Partikel zur Verwendung nach Anspruch 6, wobei die Krebszellen ausgewählt sind aus der Gruppe bestehend aus: primären oder metastasierenden Dickdarmkrebszellen, Gehirntumorzellen, Lungenkrebszellen, Pankreaskrebszellen, Nierenkrebszellen, Brustkrebszellen, Eierstockkrebszellen, Gebärmutterkrebszellen, Endometriumkrebszellen, Plattenepithelkrebszellen, Melanomkrebszellen und Prostatakrebszellen.

8. Partikel zur Verwendung nach Anspruch 1, wobei das Gewebe oder die Zellpopulation extrakorporal bestrahlt wird.

9. Partikel zur Verwendung nach Anspruch 6, wobei das Zielgewebe oder die Zielzellpopulation zirkulierende Tumorzellen oder Blutzellen sind.

10. Partikel zur Verwendung nach Anspruch 1, wobei das High-Z-Material ein Partikel mit einem durchschnittlichen Durchmesser zwischen ungefähr 8 nm und 200 nm ist.

11. Partikel zur Verwendung nach Anspruch 1, wobei das Zielmolekül eine Affinität für einen Rezeptor hat, der in Krebszellen exprimiert wird.

12. Partikel zur Verwendung nach Anspruch 1, wobei das Zielmolekül eine Affinität für ein Ziel hat, das ausgewählt ist aus der Gruppe bestehend aus: humanem epidermalem Wachstumsfaktor-Rezeptor 2, humanem epidermalem Wachstumsfaktor-Rezeptor 3, humanem epidermalem Wachstumsfaktor-Rezeptor 4, vaskulärem endothelialem Wachstumsfaktor-Rezeptor, Folsäurerezeptor, Melanocyten-stimulierendem Hormonrezeptor, Integrin avb3, Insulin-artigem (insulin-like) Wachstumsfaktor-Rezeptor, Hepatocyt-Wachstumsfaktor-Rezeptor und basischem Fibroblast-Wachstumsfaktor-Rezeptor.

13. Partikel zur Verwendung nach Anspruch 1, wobei das Zielmolekül ausgewählt ist aus der Gruppe bestehend aus: einem Antikörper für den Zielrezeptor; einem Peptid, Protein, Aptamer, Oligomer oder niedermolekularem Molekül mit Affinität für den Zielrezeptor; und Kombinationen davon.

14. Partikel zur Verwendung nach Anspruch 1, wobei das Zielmolekül ausgewählt ist aus der Gruppe bestehend aus: Cetuximab, Herceptin, Folsäure, Melanocyten-stimulierendem Hormon, cyclischem RGD und einem Analog zu cyclischem RGD.

15. Partikel zur Verwendung nach Anspruch 1, wobei die ionisierende Strahlung durchgehend durch ein Implantat aus einem Strahlung-emittierenden Stoff im Tumor oder in der Nähe des Tumors erfolgt.

## Revendications

1. Particules à Z élevé destinées à être utilisées dans un procédé pour améliorer les effets d'un rayonnement dirigé vers un tissu ou une population de cellules, où les particules à Z élevé sont des nanotiges d'or et possèdent un revêtement de surface de polyéthylène glycol (PEG) ;
les particules à Z élevé comprennent une molécule de ciblage ayant une affinité pour un tissu ciblé ou une population de cellules ciblée ;
où les particules à Z élevé sont administrées à un animal et le tissu ciblé ou la population de cellules ciblée est ensuite irradié(e) par un rayonnement ionisant ;
où les particules à Z élevé sont administrées à l'animal en une quantité suffisante pour obtenir une concentration dans le tissu ciblé ou la population de cellules ciblée inférieure à 0,05 % de métal en poids.

2. Particules destinées à être utilisées selon la revendication 1, où la molécule de ciblage entraîne l'internalisation des particules à Z élevé suite à une liaison avec le tissu ciblé ou la population de cellules ciblée.

3. Particules destinées à être utilisées selon la revendication 1, où le rayonnement ionisant est délivré en fractions sur une certaine période.

4. Particules destinées à être utilisées selon la revendication 1, où les particules à Z élevé sont administrées à l'animal une ou plusieurs fois.

5. Particules destinées à être utilisées selon la revendication 1, où le tissu ciblé ou la population de cellules ciblée est irradié(e) au sein de l'animal.

6. Particules destinées à être utilisées selon la revendication 1, où le tissu ciblé ou la population de cellules ciblée comprend des cellules cancéreuses.

7. Particules destinées à être utilisées selon la revendication 6, où les cellules cancéreuses sont sélectionnées dans le groupe consistant en : des cellules du cancer colorectal, des cellules du cancer du cerveau, des cellules du cancer du poumon, des cellules du cancer du pancréas, des cellules du cancer du rein, des cellules du cancer du sein, des cellules du cancer de l'ovaire, des cellules du cancer de l'utérus, des cellules du cancer de l'endomètre, des cellules cancéreuses squameuses, des cellules cancéreuses du mélanome, et des cellules du cancer de la prostate primaires ou métastatiques.

8. Particules destinées à être utilisées selon la revendication 1, où le tissu ou la population de cellules est irradié(e) de manière extracorporelle.

9. Particules destinées à être utilisées selon la revendication 6, où le tissu ciblé ou la population de cellules ciblée est des cellules tumorales circulantes ou des cellules sanguines.

10. Particules destinées à être utilisées selon la revendication 1, où le matériel à Z élevé est une particule ayant un diamètre moyen compris entre approximativement 8 nm et 200 nm.

11. Particules destinées à être utilisées selon la revendication 1, où la molécule de ciblage possède une affinité pour un récepteur exprimé dans des cellules cancéreuses.

12. Particules destinées à être utilisées selon la revendication 1, où la molécule de ciblage possède une affinité pour une cible sélectionnée dans le groupe consistant en : le récepteur 2 du facteur de croissance épidermique humain, le récepteur 3 du facteur de croissance épidermique humain, le récepteur 4 du facteur de croissance épidermique humain, le récepteur du facteur de croissance de l'endothélium vasculaire, le récepteur de l'acide folique, le récepteur de l'hormone stimulant les mélanocytes, l'intégrine avb3, le récepteur du facteur de croissance analogue à l'insuline, le récepteur du facteur de croissance des hépatocytes, et le récepteur du facteur de croissance des fibroblastes basique.

13. Particules destinées à être utilisées selon la revendication 1, où la molécule de ciblage est sélectionnée dans le groupe consistant en : un anticorps dirigé contre le récepteur cible ; un peptide, une protéine, un aptamère, un oligomère, ou une petite molécule ayant une affinité pour le récepteur cible ; et des combinaisons de ceux-ci.

14. Particules destinées à être utilisées selon la revendication 1, où la molécule de ciblage est sélectionnée dans le groupe consistant en : le cétuximab, l'herceptin, l'acide folique, l'hormone stimulant les mélanocytes, un peptide RGD cyclique, et un analogue de peptide RGD cyclique.

15. Particules destinées à être utilisées selon la revendication 1, où le rayonnement ionisant est délivré en continu par un implant, dans ou à proximité de la tumeur, d'une substance émettant un rayonnement.
